# EUROPEAN PATENT APPLICATION

(11) **EP 4 643 848 A1**
(43) Date of publication of application: **05.11.2025**
(21) Application number: 23910857.4
(22) Date of filing: 28.12.2023
(51) Int. Cl.: A61K 9/107, A61K 31/165, A61K 47/10, A61P 25/02

(54) **CAPSAICIN EMULSION COMPOSITION, PREPARATION METHOD THEREFOR, AND USE THEREOF**

(30) Priority: 29.12.2022 CN 202211717952
(71) Applicant: Nanjing Delova Biotech Co. Ltd., Nanjing, Jiangsu 210000 (CN)
(72) Inventor: WANG, Qingsong, Nanjing, Jiangsu 210000 (CN); YANG, Yibo, Nanjing, Jiangsu 210000 (CN); CHEN, Hailiang, Nanjing, Jiangsu 210000 (CN)
(74) Representative: Gille Hrabal Partnerschaftsgesellschaft mbB Patentanwälte
(86) International application number: PCT/CN2023/142863
(87) International publication number: WO 2024/140941

(57) **Abstract**

A capsaicin emulsion composition, a preparation method therefor, and the use thereof. More particularly, the emulsion composition comprises capsaicin, a water-soluble organic solvent, water and an emulsifier. In the emulsion, the water-soluble organic solvent shows a unique property of an oil phase; the oil phase mainly consists of an active component and the water-soluble organic solvent, and the pharmaceutically active component is mainly distributed in the oil phase. Therefore, the capsaicin emulsion composition has a greatly improved drug loading capacity which can be up to 20%, and further can deliver more effective drugs to the skin in short time and remarkably relieve the burning sting pain of capsaicin. Thus, the capsaicin emulsion composition is animal-tolerant and has prospects for clinical application.

## Description

The present application claims priority to Chinese Patent Application No. 202211717952.6 filed with China National Intellectual Property Administration on December 29, 2022 and entitled "CAPSAICIN EMULSION COMPOSITION, PREPARATION METHOD THEREFOR, AND USE THEREOF", which is incorporated herein by reference in its entirety.

### TECHNICAL FIELD

The present disclosure relates to the field of pharmaceutical formulations, and particularly to an emulsion composition of capsaicin, a preparation method therefor, and use thereof.

### BACKGROUND

The International Association for the Study of Pain (IASP) defines neuropathic pain (NP) as "pain caused by a lesion or disease of the somatosensory system". Neuropathic pain is not a single disease but rather a syndrome resulting from many different diseases and injuries, characterized by a series of symptoms and signs, and encompassing more than 100 clinical diseases. Based on the anatomical location of the lesion or disease, neuropathic pain can be classified into peripheral neuropathic pain (pNP) and central neuropathic pain, with peripheral neuropathic pain being more common in clinical practice.

Painful diabetic peripheral neuropathy (PDPN) and postherpetic neuralgia (PHN) are both common types of peripheral neuropathic pain, and the prevalence of both PDPN and PHN increases gradually with age. In addition to the two conditions mentioned above, common syndromes of peripheral neuropathic pain also include postoperative or post-traumatic peripheral neuropathic pain, drug-induced (e.g., chemotherapy-induced) peripheral neuropathic pain, cancer pain, trigeminal neuralgia, and glossopharyngeal neuralgia.

At present, there is no specific medicine available for treating peripheral neuropathic pain. Most treatments involve psychiatric drugs that have been repurposed for the indication of peripheral neuropathic pain. Clinicians and patients are still unsatisfied with the therapeutic efficacy of existing drugs, because their pain-relieving effects are unsatisfactory, and some of the patients require nerve blocks, nerve destruction, spinal cord electrical stimulation, etc. In addition, peripheral neuropathic pain is a chronic pain that requires long-term medication, and most of the conditions require multiple daily oral and systemic administrations. This is associated with central nervous system adverse effects, and some of the drugs require dose titration. Therefore, there is significant room for improvement in the safety and medication adherence of drugs used to treat peripheral neuropathic pain.

In 2009, the FDA and the European Union approved Qutenza^{®}, the first and only capsaicin prescription drug to date, for use in the treatment of neuropathic pain including PHN and PDPN. Qutenza^{®} is a topical skin patch that requires application for only 30 to 60 minutes, with a single application providing therapeutic efficacy lasting for 2 to 3 months. Additionally, it avoids the central nervous system adverse effects associated with the oral administration of psychiatric drugs, thereby greatly improving patient safety and medication adherence. Compared to the current commercially available over-the-counter drugs with low capsaicin concentrations (not exceeding 0.25%), Qutenza^{®} contains high-concentration (8%) capsaicin, allowing it to deliver a greater amount of effective medication to the skin within a short period of time, thereby ensuring therapeutic efficacy.

In 2022, the applicant disclosed in CN115006379A a developed composition containing a capsaicin film. The composition exists as a viscous liquid at room temperature and can be applied according to the shape of the lesion site. After application, it can quickly form a thin, easily removable film on the surface of the skin. The *in vitro* transdermal rate of this film agent is comparable to that of Qutenza^{®}, while offering greater convenience in use. However, this composition shares the same issue as Qutenza^{®}, i.e., it can cause severe burning sting pain (BSP) upon contact with the skin. In a safety evaluation test conducted on miniature pigs, the test subjects exhibited severe intolerance, including rubbing, trembling, vocalizing in distress, etc., which ultimately made it impossible to continue the test.

BSP is a unique property of capsaicin. In order to overcome capsaicin-induced BSP, analgesic pretreatment is usually required. For example, during toxicological studies of Qutenza^{®}, in order to enable miniature pigs to tolerate a high dose of Qutenza^{®} (applied over an area of 600 cm²), a local anesthetic needs to be applied to the skin 60 to 85 minutes before the Qutenza^{®} application, and Demerol^{™} (meperidine) should be injected intramuscularly at a dose of 10 mg/kg at the same time; one hour after the application, the same dose of Demerol^{™} should be administered. In actual patient use, Qutenza^{®} cannot be directly applied to the patient's lesion site, but a local anesthetic (e.g., lidocaine and prilocaine compound creams) needs to be applied to the patient's lesion site 60 min in advance for analgesic pretreatment.

Current commercially available capsaicin creams as over-the-counter drugs generally have a strength of no more than 0.25%. These low-concentration capsaicin formulations are typically used for relieving joint and muscle pain, shoulder soreness, lower back pain, etc., and need to be administrated multiple times a day. They cannot be used for treating neuropathic pain, let alone achieve a single-dose effect that can last for 2 to 3 months. This is because the mechanism by which capsaicin treats neuropathic pain is that the C-fibers, the nerves responsible for pain sensation, are desensitized and degenerated by capsaicin, so the pain can be eliminated or significantly reduced within a few weeks. However, this desensitization and degeneration effect occurs only when a sufficient amount of capsaicin is delivered to the skin and the concentration of capsaicin in the C-fibers is high enough. Therefore, for capsaicin formulations used to treat neuropathic pain, it is crucial to ensure high transdermal efficiency of the products. For example, the product development of Qutenza^{®} explicitly states that the design concept is to deliver as much medication as possible to the skin within a short period of time.

Since capsaicin has low solubility in commonly used oil phases in creams, such as paraffin, vaseline, silicone oil, liquid paraffin, cottonseed oil, vegetable oil, volatile oil, cetyl alcohol, stearyl alcohol, stearic acid, refined lanolin, beeswax, etc., the resulting cream formulations have a low drug-loading capacity and a low transdermal rate.

Therefore, there is an urgent clinical need to develop a novel capsaicin topical skin formulation that is less irritating, can deliver an effective dose within a short period of time, and offers greater flexibility and convenience in administration.

### SUMMARY

In order to address the technical issues present in the prior art, in one aspect, the present application provides an emulsion composition of capsaicin, which comprises:
a. capsaicin;
b. a water-soluble organic solvent;
c. water; and
d. an emulsifier.

According to an embodiment of the present disclosure, a mass percentage of capsaicin in the emulsion composition ranges from 0.01% to 20%, preferably 0.01% to 12%, preferably 0.25% to 12%, preferably 0.25% to 10%, preferably 0.25% to 8%, preferably 0.25% to 6%, preferably 1% to 6%, preferably 2% to 6%, and more preferably 2% to 4%, and an example thereof may be 0.25%, 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10%.

According to an embodiment of the present disclosure, a mass ratio of capsaicin to the water-soluble organic solvent ranges from 1:30 to 1:0.1, preferably 1:15 to 1:0.5, and more preferably 1:10 to 1:1, and an example thereof may be 1:30, 1:29.5, 1:29, 1:28.5, 1:28, 1:27.5, 1:27, 1:26.5, 1:26, 1:25.5, 1:25, 1:24.5, 1:24, 1:23.5, 1:23, 1:22.5, 1:22, 1:21.5, 1:21, 1:20.5, 1:20, 1:19.5, 1:19, 1:18.5, 1:18, 1:17.5, 1:17, 1:16.5, 1:16, 1:15.5, 1:15, 1:14.5, 1:14, 1:13.5, 1:13, 1:12.5, 1:12, 1:11.5, 1:11, 1:10.5, 1:10, 1:9.5, 1:9, 1:8.5, 1:8, 1:7.5, 1:7, 1:6.5, 1:6, 1:5.5, 1:5, 1:4.5, 1:4, 1:3.5, 1:3, 1:2.5, 1:2, 1:1.5, 1:1, 1:0.5, or 1:0.1. According to an embodiment of the present disclosure, for the ternary phase system of capsaicin, the water-soluble organic solvent, and water in the emulsion composition: stock solutions are prepared with mass ratios of capsaicin to water-soluble organic solvent ranging from 1:20 to 1:0.1; equal amounts of the stock solutions are separately taken, and different amounts of water are added; the resulting mixtures are shaken, then left to stand, and observed for physical phenomena; based on the phenomena of clarification, liquid-liquid phase separation, and precipitation, a ternary phase diagram of capsaicin, the water-soluble organic solvent, and water is drawn; and the amounts of capsaicin, the water-soluble organic solvent, and water used in the emulsion composition are selected from a range within the liquid-liquid phase separation region.

According to an embodiment of the present disclosure, for the ternary phase system of capsaicin, the water-soluble organic solvent, and water in the emulsion composition: stock solutions are prepared with mass ratios of capsaicin to water-soluble organic solvent ranging from 1:15 to 1:0.5; equal amounts of the stock solutions are separately taken, and different amounts of water are added; the resulting mixtures are shaken, then left to stand, and observed for physical phenomena; based on the phenomena of clarification, liquid-liquid phase separation, and precipitation, a ternary phase diagram of capsaicin, the water-soluble organic solvent, and water is drawn; and the amounts of capsaicin, the water-soluble organic solvent, and water used in the emulsion composition are selected from a range within the liquid-liquid phase separation region.

According to an embodiment of the present disclosure, for the ternary phase system of capsaicin, the water-soluble organic solvent, and water in the emulsion composition: stock solutions are prepared with mass ratios of capsaicin to water-soluble organic solvent ranging from 1:10 to 1:1; equal amounts of the stock solutions are separately taken, and different amounts of water are added; the resulting mixtures are shaken, then left to stand, and observed for physical phenomena; based on the phenomena of clarification, liquid-liquid phase separation, and precipitation, a ternary phase diagram of capsaicin, the water-soluble organic solvent, and water is drawn; and the amounts of capsaicin, the water-soluble organic solvent, and water used in the emulsion composition are selected from a range within the liquid-liquid phase separation region.

According to an embodiment of the present disclosure, for the ternary phase system of capsaicin, the water-soluble organic solvent, and water in the emulsion composition: stock solutions are prepared with mass ratios of capsaicin to water-soluble organic solvent being 1:1, 1:2, 1:3, 1:4, 1:5, 1:6, 1:7, 1:8, 1:9, 1:10, 1:11, 1:12, 1:13, 1:14, 1:15, 1:16, 1:17, 1:18, 1:19, and 1:20; equal amounts of the stock solutions are separately taken, and different amounts of water are added with mass ratios of water to capsaicin being 0.5:1, 1:1, 2:1, 3:1, 4:1, 5:1, 6:1, 7:1, 8:1, 9:1, 10:1, 11:1, 12:1, 13:1, 14:1, and 15:1; the resulting mixtures are shaken, then left to stand, and observed for physical phenomena; based on the phenomena of clarification, liquid-liquid phase separation, and precipitation, a ternary phase diagram of capsaicin, the water-soluble organic solvent, and water is drawn; and the amounts of capsaicin, the water-soluble organic solvent, and water used in the emulsion composition are selected from a range within the liquid-liquid phase separation region.

According to an embodiment of the present disclosure, for the ternary phase system of capsaicin, the water-soluble organic solvent, and water in the emulsion composition: stock solutions are prepared with mass ratios of capsaicin to water-soluble organic solvent being 1:1, 1:2, 1:3, 1:4, 1:5, 1:6, 1:7, 1:8, 1:9, and 1:10; equal amounts of the stock solutions are separately taken, and different amounts of water are added with mass ratios of water to capsaicin being 0.5:1, 1:1, 2:1, 3:1, 4:1, 5:1, 6:1, 7:1, 8:1, 9:1, and 10:1; the resulting mixtures are shaken, then left to stand, and observed for physical phenomena; based on the phenomena of clarification, liquid-liquid phase separation, and precipitation, a ternary phase diagram of capsaicin, the water-soluble organic solvent, and water is drawn; and the amounts of capsaicin, the water-soluble organic solvent, and water used in the emulsion composition are selected from a range within the liquid-liquid phase separation region.

According to an embodiment of the present disclosure, taking capsaicin, ethanol, and water as examples, as shown in the ternary phase diagram of capsaicin, ethanol and water samples in the accompanying drawings of the specification, the amounts of capsaicin, ethanol, and water used in the emulsion composition are selected from a range within the liquid-liquid phase separation region in the ternary phase diagram.

According to an embodiment of the present disclosure, in the emulsion composition, the three components of capsaicin, the water-soluble organic solvent, and water may form a liquid-liquid phase separation system after being shaken and left to stand.

According to an embodiment of the present disclosure, in the emulsion composition, the three components of capsaicin, the water-soluble organic solvent, and water may form a liquid-liquid phase separation system, i.e., the three components of capsaicin, the water-soluble organic solvent, and water are added in a ratio such that the ternary phase system forms a liquid-liquid phase separation system.

According to an embodiment of the present disclosure, in the emulsion composition, water is used in an amount such that the mixture of the three components of capsaicin, the water-soluble organic solvent, and water forms a liquid-liquid phase separation system.

According to an embodiment of the present disclosure, in the emulsion composition, the water-soluble organic solvent is used in an amount such that the mixture of the three components of capsaicin, the water-soluble organic solvent, and water forms a liquid-liquid phase separation system.

According to an embodiment of the present disclosure, in the emulsion composition, an emulsion can be formed by adding an emulsifier into the liquid-liquid phase separation system formed by capsaicin, the water-soluble organic solvent, and water.

According to an embodiment of the present disclosure, the water-soluble organic solvent serves as an oil phase of the emulsion composition. In some embodiments, the emulsion composition does not contain one or more oil phases commonly used in emulsions, such as paraffin, vaseline, silicone oil, liquid paraffin, cottonseed oil, vegetable oil, volatile oil, cetyl alcohol, stearyl alcohol, stearic acid, refined lanolin, beeswax, etc.

According to an embodiment of the present disclosure, in the emulsion composition, a mass ratio of water to the water-soluble organic solvent ranges from 0.2:1 to 10:1, preferably 0.2:1 to 5:1, preferably 0.5:1 to 3:1, and more preferably 0.5:1 to 2.5:1, and an example thereof may be 0.2:1, 0.5:1, 0.6:1, 0.7:1, 0.8:1, 0.9:1, 1:1, 1.1:1, 1.2:1, 1.3:1, 1.4:1, 1.5:1, 1.6:1, 1.7:1, 1.8:1, 1.9:1, 2:1, 2.1:1, 2.2:1, 2.3:1, 2.4:1, 2.5:1, 3:1, 3.5:1, 4:1, 4.5:1, 5:1, 5.5:1, 6:1, 6.5:1, 7:1, 7.5:1, 8:1, 8.5:1, 9:1, 9.5:1, or 10:1.

According to an embodiment of the present disclosure, the water-soluble organic solvent may be selected from one or more of methanol, ethanol, isopropanol, *tert*-butanol, diethylene glycol monoethyl ether, isosorbide dimethyl ether, acetonitrile, and triethanolamine. According to an embodiment of the present disclosure, the emulsifier is selected from one or more of a nonionic emulsifier, an ionic emulsifier, a natural emulsifier, and a polymer emulsifier, wherein preferably, the nonionic emulsifier is selected from one or more of Span 40, Tween 20, Tween 80, glyceryl monostearate, poloxamer 188, and polyoxyethylene hydrogenated castor oil; the ionic emulsifier is selected from one or more of sodium dodecyl sulfate, sodium hexadecyl sulfate, sodium stearyl sulfate, and triethanolamine lauryl sulfate; the natural emulsifier is selected from one or more of soybean phosphatidylcholine and egg-phosphatidylcholine; the polymer emulsifier is selected from one or more of polyvinyl alcohols.

In some embodiments, the emulsifier is selected from one or more of Span 40, Tween 20, Tween 80, glyceryl monostearate, poloxamer 188, polyoxyethylene hydrogenated castor oil, sodium dodecyl sulfate, sodium hexadecyl sulfate, sodium stearyl sulfate, triethanolamine lauryl sulfate, soybean phosphatidylcholine, egg-phosphatidylcholine, and polyvinyl alcohol.

According to an embodiment of the present disclosure, in the emulsion composition, a mass ratio of capsaicin to the emulsifier ranges from 10:1 to 1:80, preferably 10:1 to 1:50, and more preferably 10:1 to 1:10, and an example thereof may be 10:1, 9:1, 8:1, 7:1, 6:1, 5:1, 4:1, 3:1, 2:1, 1:1, 1:80, 1:75, 1:70, 1:65, 1:60, 1:55, 1:50, 1:45, 1:40, 1:35, 1:30, 1:25, 1:20, 1:19.5, 1:19, 1:18.5, 1:18, 1:17.5, 1:17, 1:16.5, 1:16, 1:15.5, 1:15, 1:14.5, 1:14, 1:13.5, 1:13, 1:12.5, 1:12, 1:11.5, 1:11, 1:10.5, 1:10, 1:9.5, 1:9, 1:8.5, 1:8, 1:7.5, 1:7, 1:6.5, 1:6, 1:5.5, 1:5, 1:4.5, 1:4, 1:3.5, 1:3, 1:2.5, 1:2, or 1:1.5.

According to an embodiment of the present disclosure, a mass percentage of the emulsifier in the composition ranges from 0.1% to 10%, preferably 0.2% to 8%, and more preferably 0.5% to 5%, and an example thereof may be 0.2%, 0.5%, 1%, 1.5%, 2%, 2.5%, 3%, 3.5%, 4%, 4.5%, 5%, 5.5%, 6%, 6.5%, 7%, 7.5%, or 8%.

According to an embodiment of the present disclosure, wherein the composition further comprises e. a film-forming material and/or a film-forming auxiliary agent and/or a thickening agent.

In some embodiments, the film-forming material is selected from one or more of polyvinyl alcohol, polyvinylpyrrolidone, methacrylic acid copolymer, vinylpyrrolidone/vinyl acetate copolymer, ethylcellulose, hydroxypropyl methylcellulose, and sodium carboxymethyl cellulose.

In some embodiments, the film-forming auxiliary agent is selected from anhydrous calcium hydrogen phosphate.

According to an embodiment of the present disclosure, a mass percentage of the film-forming material in the composition ranges from 0.01% to 25%, preferably 2% to 20%, and more preferably 5% to 15%, and an example thereof may be 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 11%, 12%, 13%, 14%, or 15%.

According to an embodiment of the present disclosure, a mass percentage of the film-forming auxiliary agent in the composition ranges from 0.01% to 25%, preferably 2% to 20%, and preferably 5% to 15%, and an example thereof may be 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 11%, 12%, 13%, 14%, or 15%.

In some embodiments, the thickening agent includes, but is not limited to, one or more of sorbitol, sucrose, a film-forming material, et al.

According to an embodiment of the present disclosure, a mass percentage of the thickening agent in the composition ranges from 0.01% to 35%, preferably 0.01% to 30%, preferably 0.01% to 25%, preferably 2% to 20%, and more preferably 5%-15%, and an example thereof may be 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 11%, 12%, 13%, 14%, or 15%. The film-forming material is as described above.

According to an embodiment of the present disclosure, wherein the composition further comprises f. a pharmaceutically acceptable preservative.

According to an embodiment of the present disclosure, the preservative is selected from one or more of methyl paraben, ethyl paraben, propyl paraben, benzoic acid, potassium sorbate, sodium benzoate, calcium sorbate, benzalkonium bromide, benzalkonium chloride, o-phenylphenol, and chlorhexidine acetate.

According to an embodiment of the present disclosure, a mass percentage of the preservative in the composition ranges from 0.01% to 0.5%, preferably 0.05% to 2%, preferably 0.08% to 1%, preferably 0.1% to 0.5%, and more preferably 0.1% to 0.2%, and an example thereof may be 0.05%, 0.1%, 0.15%, 0.2%, 0.3%, 0.4%, 0.5%, 0.6%, 0.7%, 0.8%, 0.9%, or 1%.

According to an embodiment of the present disclosure, wherein the composition further comprises g. a pharmaceutically acceptable antioxidant.

According to an embodiment of the present disclosure, the antioxidant is selected from one or more of thioglycerol, sodium hydrogen sulfite, butylated hydroxytoluene, butylated hydroxyanisole, 2,6-di-tert-butyl-p-cresol, sodium sulfite, sodium metabisulfite, sodium thiosulfate, and vitamin C and vitamin E.

According to an embodiment of the present disclosure, a mass percentage of the antioxidant in the composition ranges from 0.01% to 5%, preferably 0.05% to 1%, and preferably 0.1% to 0.5%, and an example thereof may be 0.1%, 0.15%, 0.2%, 0.3%, 0.4%, 0.5%, 0.6%, 0.7%, 0.8%, 0.9%, or 1%.

In some embodiments, the composition further comprises a fragrance agent, wherein the fragrance agent includes, but is not limited to, crystalline menthol, menthol, peppermint oil, dementholized peppermint oil, peppermint water, menthone, orange peel tincture, cinnamon oil, hesperidin, lemon oil, camphor, clove oil, eugenol, isoeugenol, chlorobutanol, urethane, nutmeg oil, allspice oil, benzyl alcohol, thyme oil, thymol, muscone, a natural perfume, a natural perfume extract, a synthetic perfume, et al.

According to an embodiment of the present disclosure, the composition is an emulsion or a cream, preferably a topical cream applied to the skin.

According to an embodiment of the present disclosure, the composition is an emulsion-based film.

The present disclosure further provides a preparation method for the emulsion composition according to any one of the embodiments described above, wherein capsaicin is first dissolved in the water-soluble organic solvent to obtain an oil phase, water is added to the oil phase, and then the emulsifier is added, followed by heating and shearing to prepare an emulsion.

The present disclosure further provides a preparation method for the emulsion composition according to any one of the embodiments described above, wherein capsaicin is first dissolved in the water-soluble organic solvent to obtain an oil phase; then the film-forming material is added to water and heated for dissolution to obtain an aqueous phase; the aqueous phase is added to the oil phase, and then the emulsifier and/or the film-forming auxiliary agent are added to prepare an emulsion-based film.

The present disclosure further provides a preparation method for the emulsion composition according to any one of the embodiments described above, wherein
the preparation method comprises the following steps:
   a1. dissolving capsaicin in the water-soluble organic solvent, adding at least one pharmaceutically acceptable preservative if necessary, adding at least one pharmaceutically acceptable antioxidant if necessary, and heating for dissolution if necessary, to obtain an oil phase;
   a2. adding the film-forming material or the film-forming auxiliary agent to water, and heating for dissolution if necessary, to obtain an aqueous phase; and
   a3. mixing the aqueous phase, the oil phase, and the emulsifier, adding at least one pharmaceutically acceptable auxiliary agent if necessary, and using a shearing device, such as a homogenizer or an emulsifying machine, for uniform mixing to obtain a sample;
or the preparation method comprises the following steps:
   b1. dissolving capsaicin in the water-soluble organic solvent, and heating for dissolution if necessary, to obtain an oil phase;
   b2. adding the film-forming material or the film-forming auxiliary agent to water, and heating for dissolution if necessary, to obtain an aqueous phase; and
   b3. mixing the aqueous phase, the oil phase, and the emulsifier, adding at least one pharmaceutically acceptable preservative if necessary, adding at least one pharmaceutically acceptable antioxidant if necessary, adding at least one pharmaceutically acceptable auxiliary agent if necessary, and using a shearing device, such as a homogenizer or an emulsifying machine, for uniform mixing to obtain a sample;
or the preparation method comprises the following steps:
   c1. dissolving capsaicin in the water-soluble organic solvent, adding the emulsifier, and heating for dissolution if necessary, to obtain
      an oil phase;
   c2. adding the film-forming material or the film-forming auxiliary agent to water, and heating for dissolution if necessary, to obtain an aqueous phase;
   c3. mixing the aqueous phase and the oil phase, adding at least one pharmaceutically acceptable preservative if necessary, adding
   at least one pharmaceutically acceptable antioxidant if necessary, and using a shearing device, such as a homogenizer or an emulsifying machine, for uniform mixing to obtain a sample; and
   c4. further adding at least one pharmaceutically acceptable auxiliary agent to obtain a sample.
The pharmaceutically acceptable auxiliary agent is a pharmaceutically acceptable thickening agent, fragrance agent, etc.

The present disclosure further provides use of the emulsion composition according to any one of the embodiments described above in the preparation of a medicament for treating pain, wherein the pain is neuropathic pain; preferably, the neuropathic pain is peripheral neuropathic pain or central neuropathic pain; preferably, the peripheral neuropathic pain is selected from painful diabetic peripheral neuropathy, postherpetic neuralgia, postoperative or post-traumatic peripheral neuropathic pain, drug-induced peripheral neuropathic pain, cancer pain, trigeminal neuralgia, and glossopharyngeal neuralgia.

The present disclosure further provides use of a water-soluble organic solvent as an oil phase in the preparation of an emulsion composition of capsaicin.

According to an embodiment of the present disclosure, the water-soluble organic solvent is selected from one or more of methanol, ethanol, isopropanol, tert-butanol, diethylene glycol monoethyl ether, isosorbide dimethyl ether, acetonitrile, and triethanolamine.

### Terms and Abbreviations

Unless otherwise stated, all numbers indicating contents, concentrations, ratios, weights, percentages, technical effects, and the like used in the specification and claims shall, in all cases, be understood as being modified by the term "about" or "approximately". "About" represents a range of ±10% of the numerical value modified thereby.

Accordingly, unless indicated to the contrary, the numerical parameters set forth in the following specification and appended claims are approximations. Unless otherwise stated, the terms used herein have the meanings that are commonly understood by those skilled in the art. It should be understood by those skilled in the art that the numerical parameters can vary depending on the desired properties and effects sought and obtained by the present disclosure, and each numerical parameter should be interpreted according to the number of significant digits and conventional rounding rules or as understood by a person skilled in the art.

Although the numerical ranges and parameters setting forth the broad scope of the present disclosure are approximations, the numerical values set forth in the specific examples are provided as precisely as possible. However, any numerical value inherently contains certain errors necessarily resulting from the standard deviation found in their respective tests or measurements. Each numerical range given in this specification will include each narrower numerical range that falls within this broader numerical range, as if such narrower numerical ranges are all explicitly written herein.

Unless otherwise stated, the definitions of excipients described in the specification and claims of the present application, including definitions thereof as examples, exemplary definitions, preferred definitions, definitions documented in tables, definitions of specific excipients provided in the examples, et al., may be arbitrarily combined and incorporated with each other. The excipients resulting from such combinations and incorporations shall fall within the scope of the present specification.

The abbreviations used in the present disclosure are defined as follows: CAP for capsaicin; EtOH for ethanol; IPA for isopropanol; DGME for diethylene glycol monoethyl ether; DMI for isosorbide dimethyl ether; PEG for polyethylene glycol; MCT for medium-chain triglyceride; GDO for glyceryl dioleate; SPC for soybean phosphatidylcholine; BHT for 2,6-di-tert-butyl-p-cresol; PVA for polyvinyl alcohol; PVP for polyvinylpyrrolidone; Span for sorbitan ester; SDS for sodium dodecyl sulfate; PVP/VA for vinylpyrrolidone/vinyl acetate copolymer; and EC for ethylcellulose.

### Beneficial Effects

(1) During the development of capsaicin emulsions, the inventors surprisingly discovered that: capsaicin has a very high solubility (greater than 250 mg/mL) in some water-soluble organic solvents, and when capsaicin, a water-soluble organic solvent, and water are mixed at an appropriate ratio, they can form a liquid-liquid phase separation system; the water-soluble organic solvent exhibits the properties of an oil phase, i.e., a stable emulsion can be prepared by adding a proper emulsifier to the liquid-liquid phase separation system (however, water-soluble solvents are usually highly miscible with water and act as demulsifiers by reducing surface tension, making it impossible to form emulsions or causing the formed emulsion-like liquid to become unstable).
(2) The capsaicin composition prepared according to the present disclosure possesses excellent stability and achieves a high drug-loading capacity, enabling the delivery of a greater amount of effective medication to the skin within a short period of time;
(3) The composition of the present disclosure is beneficial for significantly reducing capsaicin-induced BSP on the skin, eliminating the need for analgesic pretreatment before administration, and thus has good clinical application prospects.
(4) The composition of the present disclosure can be applied arbitrarily according to the shape of the skin lesion site, avoiding the need to cut a patch to match the shape of the painful area before use. Additionally, the cream is easy to wipe off, overcoming the problem of pain caused by tearing off the patch after the administration is completed.
(5) The composition of the present disclosure can further incorporate a film-forming material or a film-forming auxiliary agent to obtain an emulsion-based film; the new emulsion-based film also retains the advantages of high drug-loading capacity in the emulsion, rapid delivery of a greater amount of medication to the skin within a short period of time, and reduced burning sting pain induced by capsaicin. Moreover, the film-forming material, the film-forming auxiliary agent, and capsaicin exhibit a synergistic effect, and the film-forming performance of a product containing the film-forming material or the film-forming auxiliary agent is superior to that of a drug-free composition.

### BRIEF DESCRIPTION OF THE DRAWINGS

FIG. 1 shows a comparison of *in vitro* transdermal rate between cream A3 and Qutenza^{®} in Example 3.
FIG. 2 shows the physical phenomena observed in Example 4, including capsaicin, ethanol, and water forming a clear solution, a liquid-liquid phase separation system, and a solid precipitate.
FIG. 3 shows a ternary phase diagram of capsaicin, ethanol, and water in Example 4.
FIG. 4 shows the microscopy images of different components in the capsaicin & ethanol cream system in Example 9.
FIG. 5 shows the appearance pictures of different components in the capsaicin & ethanol cream system in Example 9.
FIG. 6 shows the *in vitro* transdermal results for cream compositions with different ethanol contents in Example 15.
FIG. 7 shows the *in vitro* transdermal results for cream compositions with different capsaicin contents in Example 16.
FIG. 8 shows an *in vitro* transdermal comparison between cream compositions and the commercially available formulation, Qutenza^{®}, as described in Example 17.
FIG. 9 shows an *in vitro* transdermal comparison between cream compositions and the commercially available formulation, Qutenza^{®}, as described in Example 18.
FIG. 10 shows the BSP scores for volunteer 1's skin following administration of creams A2 and A40 and Qutenza^{®} to the same area, as described in Example 20.
FIG. 11 shows the BSP scores for volunteer 2's skin following administration of cream A41 and Qutenza^{®} to the same area, as described in Example 20.

### DETAILED DESCRIPTION

The technical solutions of the present disclosure will be further described in detail with reference to the following specific examples. It will be appreciated that the following examples are merely exemplary illustrations and explanations of the present disclosure and should not be construed as limiting the claimed scope of the present disclosure. All techniques implemented based on the content described in this disclosure are encompassed within the claimed scope of this disclosure.

Unless otherwise stated, the starting materials and reagents used in the following examples are commercially available products or can be prepared using known methods.

### Example 1. Irritation Test of Different Capsaicin Formulations on Miniature Pigs

A capsaicin solution and a conventional cream were prepared according to the formulas listed in Table 1:
Preparation of solution A1: Capsaicin and BHT were dissolved in ethanol. Separately, polyvinyl alcohol 17-88 was dispersed in cold water and heated for dissolution. The two solutions were mixed and stirred to obtain a homogeneous and transparent capsaicin solution.

Preparation of cream A2: Capsaicin and vaseline were weighed, and then heated for dissolution to obtain an oil phase. Separately, polyvinyl alcohol 05-88 was dispersed in cold water, and then heated for dissolution, and then Span 40, methyl paraben, and propyl paraben were added and dissolved to obtain an aqueous phase. The two phases were mixed and then sheared using an IKA T25 high-speed shearing machine at 10000 rpm for 5 min in a water bath at 80 °C. Anhydrous calcium hydrogen phosphate was added as a film-forming auxiliary agent, and the mixture was sheared for an additional 3 min to obtain capsaicin cream A2. After being left to stand for 1 day, the cream exhibited crystallization phenomenon.

**Table 1. Formulas for capsaicin solution and cream**

| **Formula** | | **A1** | **A2** |
|---|---|---|---|
| **Active pharmaceutical ingredient** | Capsaicin | 4 | 4 |
| **Oil phase** | White vaseline | / | 12 |
| **Emulsifier** | Span 40 | / | 1 |
| **Film-forming agent** | PVA(05-88) | / | 12 |
| | PVA(17-88) | 12 | / |
| **Preservative** | Methyl paraben | / | 0.1 |
| | Propyl paraben | / | 0.05 |
| **Antioxidant** | BHT | 0.2 | 0 |
| **Film-forming auxiliary agent** | Anhydrous calcium hydrogen phosphate | / | 27 |
| **Solvent** | Ethanol | 40.9 | / |
| | Purified water | 42.9 | 43.85 |
| / | Total | 100 | 100 |

In accordance with the toxicological study protocol of Qutenza^{®} (Study no: 7215-113), a miniature pig animal model was selected to conduct an irritation test with different capsaicin formulations, so as to evaluate the skin irritation and tolerability of the formulations in animals. Both the solution A1 and cream A2 formulations were freshly prepared on the day of the experiment. One day before administration, the desired area on the back of the miniature pigs was shaved using a razor. During administration, each formulation was applied at 80 mg/cm² (based on the weight of the formulation). After 3 h, the film was peeled off, and the drug residue was wiped off. During this period, the pigs' behavior was observed, including but not limited to the intensity and occurrence of vocalizations, rubbing, body shaking, etc. After the drug residue was wiped off, the skin condition, such as redness, swelling, pallor, etc., was observed and recorded. The results are shown in Table 2.

**Table 2. Skin irritation, redness, swelling scores and clinical observations in miniature pigs treated with different capsaicin formulations**

| **Animal No.** | **Formulation No.** | **Application area (cm²)** | **Skin irritation evaluation** | | **Clinical observation result** | | | **Tolerance evaluation** |
|---|---|---|---|---|---|---|---|---|
| | | | **Reaction observation** | **After drug application** | **Severe reaction** | **Moderate reaction** | **Mild reaction** | |
| M04 | A1 | 400 | The skin turned red 5 min after administration, but returned to normal 20 min after the test substance was wiped off. | Mild erythema | 0.6 h to 1 h after administration / intermittent shaking and wall-rubbing, with high frequency, accompanied by reactive whining, occurring approximately once every two minutes (slightly more intense than the moderate reaction)*. | None | None | Intolerant |
| M07 | A2 | 600 | None | Normal, without erythema | None | Occurred during the period of 1.3 h to 1.6 h after administration. | Occurred during the period of 0.1 h to 1.3 h after administration, and 1.6 h to 2.8 h after administration. | Tolerant |

Severe reaction: High frequency of shaking, wall-rubbing, and reactive whining, occurring approximately once every 1-2 min.

Moderate reaction: Low frequency of shaking and wall-rubbing, occurring approximately once every few minutes.

Mild reaction: Occasional occurrence of shaking and wall-rubbing.

*During the administration period, the animal exhibited relatively severe pain-related response and rubbed off part of the applied material, indicating intolerance. Therefore, the administration was terminated early at the 1-hour mark.

Conclusion: At the same concentration of capsaicin in topical formulations, the solution formulation (A1) caused intolerance in animals and severe irritation to pig skin when applied to 400 cm²; in contrast, the cream formulation (A2) was still well tolerated in animals even when applied to 600 cm². The BSP induced by the solution was significantly stronger than that induced by the cream. After being left to stand for 1 day, a large number of capsaicin crystals were observed in A2 under a microscope, indicating that the drug-loading capacity of capsaicin in the ordinary cream was low. Therefore, the subsequent development focused on capsaicin creams with high transdermal rate and low irritation.

### Example 2. Study on Solubility of CAP in Different Pharmaceutical Solvents

About 3 g of each solvent was weighed according to Table 3, and an excess amount of capsaicin was added. The mixture was placed into a constant-temperature oscillator for oscillation for 24 h (25 °C, 200 rpm), and then centrifuged at 10000 rpm for 30 min. The supernatant was taken to measure the capsaicin content. The solubility of CAP in different solvents (different pH buffers, commonly used oil phases, and water-soluble organic solvents) was measured, with the results shown in Table 3:

**Table 3. Solubility of CAP in different solvents**

| **Solubility (shaken at 25 °C for 24 h)** | | |
|---|---|---|
| **Dissolution medium** | | **Solubility (mg/mL)** |
| **pH buffer** | pH 1.0 hydrochloric acid medium | 0.045 |
| | pH 2.0 hydrochloric acid medium | 0.052 |
| | pH 3.0 phosphate medium | 0.044 |
| | pH 4.0 acetate medium | 0.053 |
| | pH 5.0 acetate medium | 0.052 |
| | pH 6.0 phosphate medium | 0.046 |
| | pH 7.0 phosphate medium | 0.045 |
| | pH 8.0 phosphate medium | 0.042 |
| | pH 9.0 carbonate medium | 0.053 |
| | pH 10.0 carbonate medium | 0.102 |
| **Commonly used oil phase** | Soybean oil | 4 |
| | Castor oil | 36 |
| | MCT | 14 |
| | Corn oil | 5 |
| | Peanut oil | 5 |
| | Glycerol | 2 |
| | GDO | 45 |
| | Sesame oil | 4 |
| | Rapeseed oil | 5 |
| **Water-soluble organic solvent** | Ethanol | >500 |
| | PEG200 | 367 |
| | Propylene glycol | 290 |
| | Isopropanol | >500 |
| | DGME | 502 |
| | DMI | >500 |

The above results indicate that CAP had low solubility in different pH buffer media and commonly used oil phases, and had very high solubility in water-soluble organic solvents such as ethanol, PEG200, propylene glycol, isopropanol, DGME, and DMI.

### Example 3. Preparation of Ordinary Capsaicin Creams and Drug-Loading Capacity and Transdermal Studies

Three capsaicin creams were prepared according to Table 4, with reference to the classic cream base formula.

Preparation of cream A3: Capsaicin, crystalline menthol, MCT, and SPC were weighed, and then heated at 80 °C until complete dissolution to obtain an oil phase. Sucrose was weighed and then dissolved in water, and the resulting aqueous phase was added to the oil phase. The mixture was sheared using an IKA T25 high-speed shearing machine at 10000 rpm for 5 min and then homogenized 3 times using a microfluidizer at different pressures. The mixture was concentrated by rotary evaporation to remove the water until the target weight was achieved, thus obtaining a translucent cream A3.

Preparation of cream A4: Capsaicin, vaseline, stearic acid, liquid paraffin, and glyceryl monostearate were weighed, and then heated until complete dissolution to obtain an oil phase. Triethanolamine and glycerol were added to water to obtain an aqueous phase. The aqueous phase was then added to the oil phase, and the mixture was sheared using an IKA T25 high-speed shearing machine at 10000 rpm for 5 min to obtain a cream A4.

Preparation of cream A5: Capsaicin, vaseline, octadecanol, liquid paraffin, and glyceryl monostearate were weighed, and then heated until complete dissolution to obtain an oil phase. Glycerol, sodium dodecyl sulfate, and ethyl paraben were added to water to obtain an aqueous phase. The aqueous phase was then added to the oil phase, and the mixture was sheared using an IKA T25 high-speed shearing machine at 10000 rpm for 5 min to obtain a cream A5.

**Table 4. Preparation of capsaicin-containing creams using classic cream base formula**

| **Formula** | | **A3** | **A4** | **A5** |
|---|---|---|---|---|
| **Active pharmaceutical ingredient** | **Capsaicin** | 1.52 | 2 | 2 |
| **Oil phase** | **MCT** | 21.7 | / | / |
| | **Vaseline** | / | 5 | 10 |
| | **Octadecanol** | / | / | 6.5 |
| | **Stearic acid** | / | 12 | / |
| | **Liquid paraffin** | / | 5 | 7.5 |
| **Thickening agent** | **Sucrose** | 32.6 | / | / |
| **Emulsifier** | **SPC** | 21.7 | / | / |
| | **Glyceryl monostearate** | / | 3 | 4 |
| **Fragrance agent** | **Crystalline menthol** | 0.65 | / | / |
| **Aqueous phase** | **Glycerol** | / | 5 | 6.5 |
| | **Triethanolamine** | / | 0.5 | / |
| | **Sodium dodecyl sulfate** | / | / | 1.25 |
| | **Ethyl paraben** | / | / | 0.1 |
| **Solvent** | **Water** | 21.83 | 67.5 | 62.15 |
| **Total** | | 100 | 100 | 100 |
| **Stability** | | **Crystallization occurred after 3 months of long-term standing** | **Crystallization occurred after 7 days** | **Crystallization occurred after 3 days** |

As can be seen from Table 4, the capsaicin cream samples prepared using the conventional cream base had low drug-loading capacity and were unstable; crystallization occurred in A3 after 3 months of standing, in A4 after 7 days of standing, and in A5 after 3 days of standing.

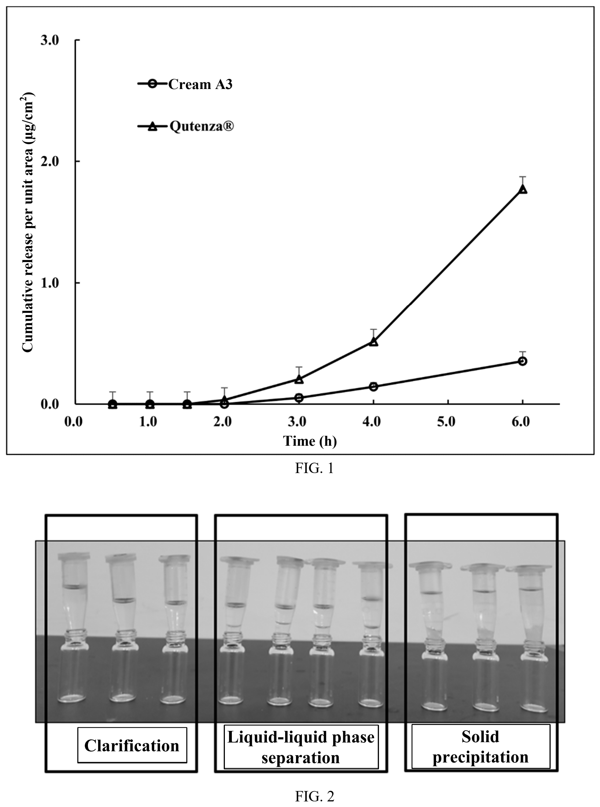

Pig skin was used as the *ex vivo* skin, and the Franz diffusion cell method was carried out to compare the *in vitro* transdermal performance of the ordinary capsaicin A3 (non-crystallized) and Qutenza^{®}. The receiving medium was 10% ethanol, the rotation speed was 200 rpm, the experimental temperature was 32 °C, and the drug dose was 300 mg. At 0.5 h, 1 h, 1.5 h, 2 h, 3 h, 4 h, and 6 h, samples of 1 mL were taken and then immediately supplemented with the same temperature and volume of medium. Each experimental group was conducted in parallel with four replicates. The *in vitro* transdermal results are shown in FIG. 1. The results show that the capsaicin cream prepared through the conventional cream base had a low drug-loading capacity and its *in vitro* transdermal rate was significantly slower than that of Qutenza^{®}. Qutenza^{®} is designed to deliver more medication to the skin within a short period of time (no more than 1 h). The transdermal rate of the conventionally prepared cream was lower than that of Qutenza^{®}, which may affect the efficacy in clinical use.

### Example 4. Study on Physical Phenomena of Mixtures of CAP-Ethanol-Water at Different Ratios

During the experimental investigation of the capsaicin solubility, it was unexpectedly discovered that capsaicin, ethanol, and water can form a liquid-liquid phase separation phenomenon. In order to comprehensively understand the phase behavior of the ternary composition, the ternary phase region for the liquid-liquid phase separation system formed by capsaicin, ethanol and water at different ratios was further investigated, and an in-depth study was conducted using a ternary phase diagram.

### 4.1. Physical phenomena of CAP:EtOH = 1:1 in different proportions of water

A stock solution was prepared with a mass ratio of CAP:EtOH = 1:1. Then, 0.5 g samples of the stock solution were taken and respectively added into PE tubes, and different amounts of water (based on mass ratio of water to CAP) were added according to Table 5. The ternary mixtures were shaken, and their physical phenomena were observed at 0 h and 24 h after standing. The results are shown in Table 5.

**Table 5. Physical phenomena of CAP:EtOH = 1:1 in different proportions of water**

| **No.** | **Mass ratio** | | **Physical phenomenon** | |
|---|---|---|---|---|
| | **CAP:EtOH in stock solution** | **Water** | **0h** | **24h** |
| 1 | 1:1 | 0.5 | Clarification | Clarification |
| 2 | | 0.65 | Liquid-liquid phase separation | Liquid-liquid phase separation |
| 3 | | 0.8 | Liquid-liquid phase separation | Liquid-liquid phase separation |
| 4 | | 0.9 | Liquid-liquid phase separation | Liquid-liquid phase separation |
| 5 | | 1 | Liquid-liquid phase separation | Liquid-liquid phase separation |
| 6 | | 1.5 | Liquid-liquid phase separation | Liquid-liquid phase separation |
| 7 | | 2 | Liquid-liquid phase separation | Liquid-liquid phase separation |
| 8 | | 2.5 | Liquid-liquid phase separation | Precipitation |
| 9 | | 2.8 | Liquid-liquid phase separation | Precipitation |
| 10 | | 3 | Precipitation | Precipitation |
| 11 | | 4 | Precipitation | Precipitation |
| 12 | | 8 | Precipitation | Precipitation |

The results indicate that when the mass ratio of CAP:EtOH was 1:1 and the mass ratio of water:EtOH was 0.5:1, the mixture was in a clear state; when the mass ratio of water:EtOH was within the range of (0.65-2):1, the mixture could form a liquid-liquid phase separation state both at 0 h and 24 h after standing; when the mass ratio of water:EtOH was higher than 2.5:1, crystallization occurred. The ternary phenomenon pictures for the different states are shown in FIG. 2.

### 4.2. Physical phenomena of CAP:EtOH = 1:5 in different proportions of water

A stock solution was prepared with a mass ratio of CAP:EtOH = 1:5. Then, 0.5 g samples of the stock solution were taken and respectively added into PE tubes, and different amounts of water (based on mass ratio of water to CAP) were added according to Table 6. The ternary mixtures were shaken, and their physical phenomena were observed at 0 h and 24 h after standing. The results are shown in Table 6.

**Table 6. Physical phenomena of CAP:EtOH (1:5) in different proportions of water**

| **No.** | **Mass ratio** | | **Physical phenomenon** | |
|---|---|---|---|---|
| | **CAP:EtOH in stock solution** | **Water** | **0h** | **24h** |
| 1 | 1:5 | 2 | Clarification | Clarification |
| 2 | | 4 | Clarification | Clarification |
| 3 | | 5 | Liquid-liquid phase separation | Liquid-liquid phase separation |
| 4 | | 6 | Liquid-liquid phase separation | Liquid-liquid phase separation |
| 5 | | 7 | Liquid-liquid phase separation | Liquid-liquid phase separation |
| 6 | | 7.5 | Liquid-liquid phase separation | Liquid-liquid phase separation |
| 7 | | 7.8 | Liquid-liquid phase separation | Liquid-liquid phase separation |
| 8 | | 8 | Liquid-liquid phase separation | Liquid-liquid phase separation |
| 9 | | 9 | Liquid-liquid phase separation | Liquid-liquid phase separation |
| 10 | | 10 | Liquid-liquid phase separation | Liquid-liquid phase separation |
| 11 | | 11 | Liquid-liquid phase separation | Liquid-liquid phase separation |
| 12 | | 12 | Liquid-liquid phase separation | Precipitation |
| 13 | | 13 | Liquid-liquid phase separation | Precipitation |
| 14 | | 14 | Liquid-liquid phase separation | Precipitation |
| 15 | | 15 | Liquid-liquid phase separation | Precipitation |
| 16 | | 16 | Liquid-liquid phase separation | Precipitation |
| 17 | | 17 | Liquid-liquid phase separation | Precipitation |
| 18 | | 18 | Precipitation | Precipitation |
| 19 | | 20 | Precipitation | Precipitation |
| 20 | | 24 | Precipitation | Precipitation |
| 21 | | 32 | Precipitation | Precipitation |

The results indicate that when the mass ratio of CAP:EtOH was 1:5 and the mass ratio of water:EtOH was within the range of (1-2.2):1, the mixture could form a liquid-liquid phase separation state both at 0 h and 24 h after standing.

### 4.3. Physical phenomena of CAP:EtOH = 1:10 in different proportions of water

A stock solution was prepared with a mass ratio of CAP:EtOH = 1:10. Then, 0.5 g samples of the stock solution were taken and respectively added into PE tubes, and different amounts of water (based on mass ratio of water to CAP) were added according to Table 7. The ternary mixtures were shaken, their physical phenomena were observed at 0 h and 24 h after standing. The results are shown in Table 7.

**Table 7. Physical phenomena of CAP:EtOH = 1:10 in different proportions of water**

| **No.** | **Mass ratio** | | **Physical phenomenon** | |
|---|---|---|---|---|
| | **CAP:EtOH in stock solution** | **Water** | **0h** | **24h** |
| 1 | 1:10 | 6 | Clarification | Clarification |
| 2 | | 6.5 | Clarification | Clarification |
| 3 | | 9 | Clarification | Clarification |
| 4 | | 10 | Clarification | Clarification |
| 5 | | 11 | Clarification | Clarification |
| 6 10 | | 14 | Liquid-liquid phase separation | Liquid-liquid phase separation |
| 7 | | 18 | Liquid-liquid phase separation | Liquid-liquid phase separation |
| 8 | | 20 | Liquid-liquid phase separation | Liquid-liquid phase separation |
| 9 | | 22 | Liquid-liquid phase separation | Liquid-liquid phase separation |
| | | 25 | Liquid-liquid phase separation | Liquid-liquid phase separation |
| 11 | | 29 | Precipitation | Precipitation |

The results indicate that when the mass ratio of CAP:EtOH was 1:10 and the mass ratio of water:EtOH was within the range of (1.4-2.5):1, the mixture could form a liquid-liquid phase separation state both at 0 h and 24 h after standing.

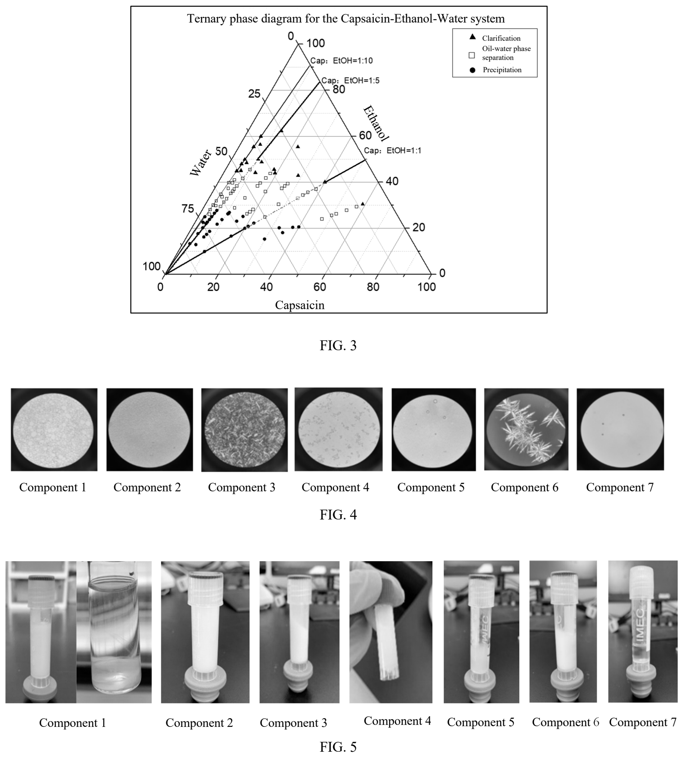

According to the protocols in Examples 4.1-4.3, the physical states of mixtures containing CAP and EtOH (at mass ratios of 1:1, 1:5, and 1:10) and different proportions of water (based on mass ratio of water to CAP) were studied at 0 h and 24 h after standing. Further, samples of CAP:EtOH:water with different mass ratios were prepared, and a ternary phase diagram was created using the Origin software, as shown in FIG. 3: After 24 h of standing, CAP:EtOH:water ternary mixtures with different ratios could form a liquid-liquid phase separation state region, as indicated in the ternary phase diagram by the marked oil-water phase separation region.

### Example 5. Study on Physical Phenomena of Mixtures of CAP-Other Water-Soluble Solvents-Water

### 5.1. Physical phenomena of CAP:PEG200 = 1:3 in different proportions of water

A stock solution was prepared with a mass ratio of CAP:PEG200 = 1:3. Then, 0.5 g samples of the stock solution were taken and respectively added into PE tubes, and different amounts of water (based on mass ratio of water to CAP) were added according to Table 8. The ternary mixtures were shaken, and their physical phenomena were observed at 0 h and 24 h after standing. The results are shown in Table 8.

**Table 8. Physical phenomena of CAP:PEG200 = 1:3 in different proportions of water**

| **No.** | **Mass ratio** | | **Physical phenomenon** | |
|---|---|---|---|---|
| | **CAP:PEG200 in stock solution** | **Water** | **0h** | **24h** |
| 1 | 1:3 | 0.3 | Clarification | Clarification |
| 2 | | 0.5 | Clarification | Precipitation |
| 3 | | 0.7 | Clarification | Precipitation |
| 4 | | 0.9 | Clarification | Precipitation |
| 5 | | 1 | Clarification | Precipitation |
| 6 | | 2 | Clarification | Precipitation |

The results show that when PEG200 was used as the water-soluble solvent and the mass ratio of CAP:PEG200 was 1:3, capsaicin, PEG200, and water could not form a liquid-liquid phase separation system.

### 5.2. Physical phenomena of CAP:PEG200 = 1:5 in different proportions of water

A stock solution was prepared with a mass ratio of CAP:PEG200 = 1:5. Then, 0.5 g samples of the stock solution were taken and respectively added into PE tubes, and different amounts of water (based on ratio of water to CAP) were added at a to Table 9. The ternary mixtures were shaken, and their physical phenomena were observed at 0 h and 24 h after standing. The results are shown in Table 9.

**Table 9. Physical phenomena of CAP:PEG200 = 1:5 in different proportions of water**

| **No.** | **Mass ratio** | | **Physical phenomenon** | |
|---|---|---|---|---|
| | **CAP:PEG200 in stock solution** | **Water** | **0h** | **24h** |
| 1 | 1:5 | 1 | Clarification | Precipitation |
| 2 | | 1.2 | Clarification | Precipitation |
| 3 | | 1.5 | Clarification | Precipitation |
| 4 | | 2 | Precipitation | Precipitation |

The results show that when PEG200 was used as the water-soluble solvent, increasing the mass of PEG200 failed to enable the formation of a liquid-liquid phase separation system with capsaicin, PEG200, and water.

### 5.3. Physical phenomena of CAP:propylene glycol = 1:5 in different proportions of water

A stock solution was prepared with a mass ratio of CAP:propylene glycol = 1:5. Then, 0.5 g samples of the stock solution were taken and respectively added into PE tubes, and different amounts of water (based on mass ratio of water to CAP) were added according to Table 10. The ternary mixtures were shaken, and their physical phenomena were observed at 0 h and 24 h after standing. The results are shown in Table 10.

**Table 10. Physical phenomena of CAP:propylene glycol = 1:5 in different proportions of water**

| **No.** | **Mass ratio** | | **Physical phenomenon** | |
|---|---|---|---|---|
| | **CAP:propylene glycol in stock solution** | **Water** | **0h** | **24h** |
| 1 | 1:5 | 1 | Clarification | Precipitation |
| 2 | | 1.2 | Clarification | Precipitation |
| 3 | | 1.5 | Precipitation | Precipitation |
| 4 | | 2 | Precipitation | Precipitation |

The results show that when propylene glycol was used as the water-soluble solvent, capsaicin, propylene glycol, and water also failed to form a liquid-liquid phase separation system.

### 5.4. Physical phenomena of CAP:IPA = 1:1 in different proportions of water

A stock solution was prepared with a mass ratio of CAP:IPA = 1:1. Then, 0.5 g samples of the stock solution were taken and respectively added into PE tubes, and different amounts of water (based on mass ratio of water to CAP) were added according to Table 11. The ternary mixtures were shaken, and their physical phenomena were observed at 0 h and 24 h after standing. The results are shown in Table 11.

**Table 11. Physical phenomena of CAP:IPA = 1:1 in different proportions of water**

| **No.** | **Mass ratio** | | **Physical phenomenon** | |
|---|---|---|---|---|
| | **CAP:IPA in stock solution** | **Water** | **0h** | **24h** |
| 1 | 1:1 | 0.125 | Clarification | Clarification |
| 2 | | 0.25 | Clarification | Clarification |
| 3 | | 0.5 | Liquid-liquid phase separation | Liquid-liquid phase separation |
| 4 | | 1 | Liquid-liquid phase separation | Liquid-liquid phase separation |
| 5 | | 2 | Liquid-liquid phase separation | Liquid-liquid phase separation |
| 6 | | 4 | Liquid-liquid phase separation | Precipitation |
| 7 | | 8 | Liquid-liquid phase separation | Precipitation |

The results show that when IPA was used as the water-soluble solvent, capsaicin, IPA, and water also could form a liquid-liquid phase separation system, with the mass ratio of water:IPA being (0.5-2):1.

### 5.5. Physical phenomena of CAP:DGME = 1:1 in different proportions of water

A stock solution was prepared with a mass ratio of CAP:DGME = 1:1. Then, 0.5 g samples of the stock solution were taken and respectively added into PE tubes, and different amounts of water (based on ratio of water to CAP) were added according to Table 12. The ternary mixtures were shaken, and their physical phenomena were observed at 0 h and 24 h after standing. The results are shown in Table 12.

**Table 12. Physical phenomena of CAP:DGME = 1:1 in different proportions of water**

| **No.** | **Mass ratio** | | **Physical phenomenon** | |
|---|---|---|---|---|
| | **CAP:DGME in stock solution** | **Water** | **0h** | **24h** |
| 1 | 1:1 | 0.5 | Clarification | Clarification |
| 2 | | 1 | Liquid-liquid phase separation | Liquid-liquid phase separation |
| 3 | | 2 | Liquid-liquid phase separation | Liquid-liquid phase separation |
| 4 | | 3 | Liquid-liquid phase separation | Precipitation |
| 5 | | 4 | Liquid-liquid phase separation | Precipitation |
| 6 | | 5 | Liquid-liquid phase separation | Precipitation |
| 7 | | 6 | Liquid-liquid phase separation | Precipitation |
| 8 | | 7 | Liquid-liquid phase separation | Precipitation |
| 9 | | 8 | Liquid-liquid phase separation | Precipitation |
| 10 | | 9 | Liquid-liquid phase separation | Precipitation |
| 1 1 | | 10 | Liquid-liquid phase separation | Precipitation |

The results show that when DGME was used as the water-soluble solvent, capsaicin, DGME, and water also could form a liquid-liquid phase separation system, with the mass ratio of water: DGME being (1-2):1.

### 5.6. Physical phenomena of CAP:DMI = 1:3 in different proportions of water

A stock solution was prepared with a mass ratio of CAP:DMI = 1:3. Then, 0.4 g samples of the stock solution were taken and respectively added into PE tubes, and different amounts of water (based on mass ratio of water to CAP) were added according to Table 13. The ternary mixtures were shaken, and their physical phenomena were observed at 0 h and 24 h after standing. The results are shown in Table 13.

**Table 13. Physical phenomena of CAP:DMI = 1:3 in different proportions of water**

| **No.** | **Mass ratio** | | **Physical phenomenon** | |
|---|---|---|---|---|
| | **CAP:DMI in stock solution** | **Water** | **0h** | **24h** |
| 1 | 1:3 | 0.5 | Clarification | Clarification |
| 2 | | 1 | Liquid-liquid phase separation | Liquid-liquid phase separation |
| 3 | | 2 | Liquid-liquid phase separation | Liquid-liquid phase separation |
| 4 | | 4 | Liquid-liquid phase separation | Liquid-liquid phase separation |
| 5 | | 6 | Liquid-liquid phase separation | Liquid-liquid phase separation |
| 6 | | 8 | Liquid-liquid phase separation | Precipitation |
| 7 | | 10 | Liquid-liquid phase separation | Precipitation |

The results show that when DMI was used as the water-soluble solvent, capsaicin, DMI, and water also could form a liquid-liquid phase separation system, with the mass ratio of water:DMI being (0.3-2):1.

### Example 6. Comparative Study on Physical Phenomena of Mixtures of Different Active Ingredient-Water-Soluble Organic Solvent-Water

### 6.1. Physical phenomena of bupivacaine:EtOH = 1:2 in different proportions of water

A stock solution was prepared with a mass ratio of bupivacaine:EtOH = 1:2. Then, 0.5 g samples of the stock solution were taken and respectively added into PE tubes, and different amounts of water (based on ratio of water to bupivacaine) were added according to Table 14. The ternary mixtures were shaken and observed at 0 h and 24 h after standing. No liquid-liquid phase separation was observed. The results are shown in Table 14.

**Table 14. Physical phenomena of bupivacaine:EtOH (= 1:2) in different proportions of water**

| **No.** | **Mass ratio** | **Physical phenomenon** | | |
|---|---|---|---|---|
| | **Bupivacaine:EtOH in stock solution** | **Water** | **0h** | **24h** |
| 1 | 1:2 | 0.375 | Clarification | Clarification |
| 2 | | 0.75 | Clarification | Clarification |
| 3 | | 1.5 | Precipitation | Precipitation |
| 4 | | 4 | Precipitation | Precipitation |
| 5 | | 6 | Precipitation | Precipitation |
| 6 | | 9 | Precipitation | Precipitation |
| 7 | | 12 | Precipitation | Precipitation |
| 8 | | 15 | Precipitation | Precipitation |

The results show that when the analgesic was bupivacaine, bupivacaine, ethanol, and water failed to form a liquid-liquid phase separation system.

### 6.2. Physical phenomena of ropivacaine:EtOH = 1:10 in different proportions of water

A stock solution was prepared with a mass ratio of ropivacaine:EtOH = 1:10. Then, 0.5 g samples of the stock solution were taken and respectively added into PE tubes, and different amounts of water (based on ratio of water to ropivacaine) were added according to Table 15. The ternary mixtures were shaken and observed at 0 h and 24 h after standing. No liquid-liquid phase separation was observed. The results are shown in Table 15.

**Table 15. Physical phenomena of ropivacaine:EtOH (= 1:10) in different proportions of water**

| **No.** | **Mass ratio** | | **Physical phenomenon** | |
|---|---|---|---|---|
| | **Ropivacaine:EtOH in stock solution** | **Water** | **0h** | **24h** |
| 1 | 1:10 | 0.25 | Clarification | Clarification |
| 2 | | 0.5 | Clarification | Clarification |
| 3 | | 1 | Clarification | Clarification |
| 4 | | 1.375 | Clarification | Clarification |
| 5 | | 2.75 | Clarification | Clarification |
| 6 | | 5.5 | Precipitation | Precipitation |
| 7 | | 6 | Precipitation | Precipitation |
| 8 | | 8 | Precipitation | Precipitation |
| 9 | | 10 | Precipitation | Precipitation |

The results show that when the analgesic was ropivacaine, ropivacaine, ethanol, and water failed to form a liquid-liquid phase separation system.

### Example 7. Study on Composition of Liquid-Liquid Phase Separation System in Ternary Phase Diagram for Capsaicin-Ethanol-Water System

Samples were prepared by selecting points within the liquid-liquid phase separation region of the ternary phase diagram for capsaicin, ethanol and water in Example 4. According to the mass ratios listed in Table 16, capsaicin, ethanol, and water were weighed into 10 mL PE tubes. Each mixture was uniformly mixed and left to stand for 24 h, resulting in the formation of a liquid-liquid phase separation system. The system was then centrifuged at 10000 rpm for 30 min and separated using a pipette. The contents of CAP, ethanol and water in the upper-layer aqueous phase solution and the lower-layer oil phase solution were measured separately, and the results are shown in Table 16.

**Table 16. Capsaicin distribution in upper and lower layers of capsaicin-ethanol-water samples with different ratios 24 h after standing for liquid-liquid phase separation system formation**

| Sample No. | | Sample 1 | Sample 2 | Sample 3 |
|---|---|---|---|---|
| Mass ratio (w:w:w) | Capsaicin | 1 | 1 | 1 |
| | Ethanol | 1 | 1 | 5 |
| | Water | 0.8 | 1.6 | 9 |
| Upper layer (%) | Capsaicin | 5.5 | 0.6 | 0.9 |
| | Ethanol | 43.1 | 32.2 | 34.5 |
| | Water | 51.4 | 67.2 | 64.6 |
| Lower layer (%) | Capsaicin | 54.3 | 73.4 | 69.7 |
| | Ethanol | 31.9 | 19.4 | 21.8 |
| | Water | 15.4 | 8.0 | 8.5 |
| **Proportion of capsaicin in the lower layer to the total amount of drug added (%)** | | **94.0** | **98.8** | **88.1** |

The results show that after the formation of liquid-liquid phase separation system by capsaicin, ethanol and water, capsaicin was primarily distributed in the lower-layer oil phase solution, and the oil phase was mainly composed of capsaicin and ethanol, which is beneficial for reducing capsaicin-induced BSP after topical administration.

### Example 8. Investigation of Capsaicin Distribution in Liquid-Liquid Phase Separation System of Cream Composition

In order to investigate the effect of other excipients in the cream on the distribution of capsaicin in the liquid-liquid phase separation system, topical formulation excipients, such as an emulsifier, a film-forming auxiliary agent, an antioxidant, a thickening agent, a preservative, and a fragrance agent, were added to the liquid-liquid phase separation system formed by capsaicin, ethanol and water to prepare a cream sample. The formula is shown in Table 17. The cream was centrifuged at 10000 rpm for 60 min, and then the upper-layer oil phase solution and the lower-layer aqueous phase solution were separated using a pipette. The content of CAP in each phase was measured separately.

**Table 17. Formula table of capsaicin & ethanol cream sample**

| **Component** | **Formula** | **Mass percentage/%** |
|---|---|---|
| **Active ingredient** | Capsaicin | 4.45 |
| **Oil phase solvent** | Ethanol | 22.23 |
| **Emulsifier** | Span 40 | 2.22 |
| **Film-forming auxiliary agent** | Anhydrous calcium hydrogen phosphate | 20.79 |
| **Preservative** | Methyl paraben | 0.11 |
| | Propyl paraben | 0.06 |
| **Antioxidant** | 2,6-Di-*tert*-butyl-*p*-cresol | 0.33 |
| **Fragrance agent** | Crystalline menthol | 0.89 |
| **Thickening agent** | Sorbitol | 11.12 |
| **Solvent** | Purified water | 37.80 |
| Total | | 100.00 |

**Table 18. Distribution of capsaicin in different phases of capsaicin & ethanol cream sample**

| Cream | Capsaicin concentration (mg/mL) | Capsaicin mass (mg) | capsaicin proportion (%) |
|---|---|---|---|
| Oil phase | 0.401 | 33.289 | 98.5 |
| Aqueous phase | 0.005 | 0.509 | 1.5 |

It can be seen that after capsaicin, ethanol and water form a liquid-liquid phase separation system, commonly used topical formulation excipients can be added to develop cream samples, thereby reducing the proportion of capsaicin in the aqueous phase to as low as 1.5%, which is beneficial for decreasing capsaicin-induced BSP.

### Example 9. Investigation of Mechanism of Oil Phase in Capsaicin & Ethanol Composition Cream

In general, if the emulsion formula contains ethanol, ethanol will diffuse into the aqueous phase because ethanol is miscible with water. The present disclosure unexpectedly discovered that capsaicin, ethanol and water, together with an emulsifier, can form a stable emulsion system, wherein the simultaneous presence of capsaicin and ethanol is a necessary condition for forming a stable oil phase. The mechanism of its formation was studied. The formula composition is shown in Table 19, and the microscopy images and appearance pictures are shown in FIGs. 4 and 5, respectively.

As shown in the appearance and microscopy of component 1, the mixture of capsaicin, ethanol and water formed an emulsion-like solution after stirring, with distribution of droplets of varying sizes observed under the microscope; after standing, liquid-liquid phase separation occurred.

Component 2 was based on component 1, with the addition of emulsifiers Span 40 and PVA. After stirring, a white semi-solid cream was obtained, with uniformly distributed small droplets observed under the microscope. After long-term standing, the sample remained stable without phase separation or crystallization.

Component 3 was based on component 2, with the removal of ethanol. Its appearance was similar to that of component 2, but a large number of capsaicin crystals were observed under the microscope.

Component 4 was based on component 2, with the removal of capsaicin. Its appearance was similar to that of component 2, but aggregated droplets were observed under the microscope. This is because Span 40 can be dispersed in hot water under heating conditions, forming an emulsion-like solution.

Span 40 was replaced with the water-soluble surfactant polysorbate 80 (Tween 80), and a sample was prepared using the same method and observed. Component 5 was based on capsaicin, ethanol and water, with the addition of Tween 80 and PVA. It also appeared as a white semi-solid cream, with uniformly distributed small droplets observed under the microscope. After long-term standing, the sample remained stable without phase separation or crystallization.

Component 6 was based on component 5, with a reduced amount of ethanol. A large number of capsaicin crystals were observed under the microscope.

Component 7 was based on component 5, with the removal of capsaicin. It appeared as a transparent and clear sample, with no droplets observed under the microscope.

**Table 19. Appearance and microscopy of different components in the capsaicin & ethanol cream system**

| No. | Ingredient | Appearance | Microscopy | Note |
|---|---|---|---|---|
| Component 1 | Capsaicin + ethanol + water | Layered liquid | Droplets of varying sizes | Layered liquid |
| Component 2 | Capsaicin + ethanol + water + Span 40 + PVA | White semi-solid cream | Uniformly distributed small droplets | Addition of emulsifiers Span 40 and PVA to component 1 |
| Component 3 | Capsaicin + water + Span 40 + PVA | White emulsion-like | Capsaicin crystals | Removal of ethanol from component 2 |
| Component 4 | Ethanol + water + Span 40 + PVA | White emulsion-like | Aggregated droplets | Removal of capsaicin from component 2 |
| Component 5 | Capsaicin + ethanol + water + Tween 80 + PVA | White semi-solid cream | Uniformly distributed small droplets | Addition of emulsifier Tween 80 and film-forming agent PVA to component 1 |
| Component 6 | Capsaicin + water + Tween 80 + PVA | White emulsion-like | Capsaicin crystals | Removal of ethanol from component 5 |
| Component 7 | Ethanol + water + Tween 80 + PVA | Clear liquid | Without droplets and crystals | Removal of capsaicin from component 5 |

The above study shows that: components 3 and 6 did not contain ethanol, and the ordinary surfactants alone were insufficient for solubilization, leading to the precipitation of capsaicin crystals; components 4 and 7 did not contain capsaicin, and because ethanol was miscible with water, the surfactants PVA and Tween 80 were dissolved in the aqueous ethanol solution, while Span 40 was dispersed in the aqueous ethanol solution; components 2 and 5 were oil-in-water emulsion-like liquids with typical structures, formed by adding surfactants (PVA, Span 40, or Tween 80) to the ternary system of capsaicin, ethanol and water. Therefore, the formulation characteristic of the capsaicin & ethanol cream system is that capsaicin and ethanol constitute the oil phase of the cream.

### Example 10. Preparation of Cream Compositions with Capsaicin/Ethanol/Water/Different Emulsifiers

Preparation method: According to the formula composition listed in Table 20, capsaicin was first dissolved with stirring in ethanol to obtain an oil phase. Water was added to the oil phase to form a liquid-liquid phase separation state, and then an emulsifier was added. A Bead Beater device was used to prepare capsaicin emulsions containing different emulsifiers.

**Table 20. Formulas (wt%) of creams containing capsaicin/EtOH/water/different emulsifiers**

| **Formula** | | **A6** | **A7** | **A8** | **A9** | **A10** | **A11** | **A12** | **A13** | **A14** |
|---|---|---|---|---|---|---|---|---|---|---|
| **Capsaicin** | | 4 | 4 | 4 | 4 | 4 | 4 | 4 | 4 | 4 |
| **Ethanol** | | 30 | 30 | 30 | 30 | 30 | 30 | 30 | 30 | 30 |
| **Ionic emulsifier** | **SDS** | 2 | / | / | / | / | / | / | / | / |
| **Natural emulsifier** | **Lecithin** | / | 5 | / | / | / | / | / | / | / |
| **Polymer emulsifier** | **Polyvinyl alcohol** | / | / | 10 | / | / | / | / | / | / |
| **Nonionic emulsifier** | **Span 40** | / | / | / | 5 | / | / | / | / | / |
| | **Tween 20** | / | / | / | / | 5 | / | / | / | / |
| | **Tween 80** | / | / | / | / | / | 5 | / | / | / |
| | **Glyceryl monostearate** | / | / | / | / | / | / | 5 | / | / |
| | **Poloxamer 188** | / | / | / | / | / | / | / | 5 | / |
| | **Polyoxyethylene hydrogenated** | / | / | / | / | / | / | / | / | 5 |
| | **castor oil** | | | | | | | | | |
| **Purified water** | | 64 | 61 | 56 | 61 | 61 | 61 | 61 | 61 | 61 |
| **Total** | | 100 | 100 | 100 | 100 | 100 | 100 | 100 | 100 | 100 |
| **Appearance** | | Translucent emulsion | Emulsion-like, with emulsion droplets observed under the microscope | Emulsion-like, with emulsion droplets observed under the microscope | Emulsion-like, with emulsion droplets observed under the microscope | Emulsion-like, with emulsion droplets observed under the microscope | Emulsion-like, with emulsion droplets observed under the microscope | Emulsion-like, with emulsion droplets observed under the microscope | Emulsion-like, with emulsion droplets observed under the microscope | Emulsion-like, with emulsion droplets observed under the microscope |
| **Microscopic observation** | | No CAP crystals | No CAP crystals | No CAP crystals | No CAP crystals | No CAP crystals | No CAP crystals | No CAP crystals | No CAP crystals | No CAP crystals |

Preparation method: According to the formula composition listed in Table 21, capsaicin was first dissolved with stirring in DGME to obtain an oil phase. Water was added to the oil phase to form a liquid-liquid phase separation state, and then an emulsifier was added. A Bead Beater device was used to prepare capsaicin emulsions containing different emulsifiers.

**Table 21. Formulas (wt%) of creams containing capsaicin/DGME/water/different emulsifiers**

| **Formula** | | **A15** | **A16** | **A17** | **A18** | **A19** | **A20** | **A21** | **A22** | **A23** |
|---|---|---|---|---|---|---|---|---|---|---|
| **Capsaicin** | | 4 | 4 | 4 | 4 | 4 | 4 | 4 | 4 | 4 |
| **DGME** | | 30 | 30 | 30 | 30 | 30 | 30 | 30 | 30 | 30 |
| **Ionic emulsifier** | **SDS** | 10 | / | / | / | / | / | / | / | / |
| **Natural emulsifier** | **Lecithin** | / | 5 | / | / | / | / | / | / | / |
| **Polymer emulsifier** | **Polyvinyl alcohol** | / | / | 10 | / | / | / | / | / | / |
| **Nonionic emulsifier** | **Span 40** | / | / | / | 5 | / | / | / | / | / |
| | **Tween 20** | / | / | / | / | 5 | / | / | / | / |
| | **Tween 80** | / | / | / | / | / | 5 | / | / | / |
| | **Glyceryl monostearate** | / | / | / | / | / | / | 5 | / | / |
| | **Poloxamer 188** | / | / | / | / | / | / | / | 5 | / |
| | **Polyoxyethylene hydrogenated castor oil** | / | / | / | / | / | / | / | / | 5 |
| **Purified water** | | 56 | 61 | 56 | 61 | 61 | 61 | 61 | 61 | 61 |
| **Total** | | 100 | 100 | 100 | 100 | 100 | 100 | 100 | 100 | 100 |
| **Appearance** | | Translucent emulsion | Emulsion-like, with emulsion droplets observed under the microscope | Emulsion-like, with emulsion droplets observed under the microscope | Emulsion-like, with emulsion droplets observed under the microscope | Emulsion-like, with emulsion droplets observed under the microscope | Emulsion-like, with emulsion droplets observed under the microscope | Emulsion-like, with emulsion droplets observed under the microscope | Emulsion-like, with emulsion droplets observed under the microscope | Emulsion-like, with emulsion droplets observed under the microscope |
| **Microscopic observation** | | No CAP crystals | No CAP crystals | No CAP crystals | No CAP crystals | No CAP crystals | No CAP crystals | No CAP crystals | No CAP crystals | No CAP crystals |

After capsaicin, ethanol/DGME, and water form a liquid-liquid phase separation system, different types of emulsifiers can be added to prepare emulsions, which can be further developed into emulsion products.

### Example 11. Capsaicin Cream Composition

Preparation method: According to the formula composition listed in Table 22, capsaicin, an antioxidant, a preservative, and a fragrance agent were first dissolved with stirring in ethanol/DGME to obtain an oil phase. Separately, different film-forming materials were added to water, and then heated for dissolution to obtain an aqueous phase. The aqueous phase was added to the oil phase, and then an emulsifier and a thickening agent were added. A Bead Beater device or a homogenizer was used to prepare a capsaicin cream.

**Table 22. Formulas (wt%) of cream compositions containing capsaicin**

| **Formula** | | **A24** | **A25** | **A26** | **A27** | **A28** | **A29** | **A30** | **A31** | **A32** | **A33** |
|---|---|---|---|---|---|---|---|---|---|---|---|
| **Active ingredient** | **Capsaicin** | 3 | 4 | 4 | 4 | 4 | 4 | 4 | 12 | 16 | 20 |
| **Water-soluble solvent** | **Ethanol** | / | 20 | 20 | 20 | 20 | 20 | 20 | 24 | 24 | 20 |
| | **DGME** | 20 | / | / | / | / | / | / | / | / | / |
| **Emulsifier** | **span 40** | 2 | 2 | 2 | 2 | 2 | 2 | 2 | 2 | 2 | 2 |
| **Film-forming agent** | **PVA 05-88** | 15 | 15 | 15 | / | / | 15 | 15 | / | / | / |
| | **PVA 17-88** | / | / | / | / | / | / | / | 10 | 10 | 10 |
| | **PVP** | / | / | / | 15 | / | / | / | / | / | / |
| | **PVP/VA (VA64)** | / | / | / | / | 15 | / | / | / | / | / |
| **Film-forming auxiliary agent** | **Anhydrous calcium hydrogen phosphate** | / | 18.35 | / | / | / | 18.15 | 18.15 | / | / | / |
| **Thickening agent** | **Sorbitol** | 19.35 | / | 18.35 | 18.35 | 18.35 | / | / | 12 | 8 | 8 |
| **Antioxidant** | **Sodium hydrogen sulfite** | / | / | / | / | / | 0.5 | / | / | / | / |
| | **Butylated hydroxytoluene** | 0.3 | 0.3 | 0.3 | 0.3 | 0.3 | / | 0.5 | / | / | / |
| **Preservative** | **Methyl paraben** | 0.1 | 0.1 | 0.1 | 0.1 | 0.1 | 0.1 | 0.1 | / | / | / |
| | **Propyl paraben** | 0.05 | 0.05 | 0.05 | 0.05 | 0.05 | 0.05 | 0.05 | / | / | / |
| **Fragrance agent** | **Crystalline menthol** | 0.2 | 0.2 | 0.2 | 0.2 | 0.2 | 0.2 | 0.2 | / | / | / |
| **Solvent** | **Purified water** | 40 | 40 | 40 | 40 | 40 | 40 | 40 | 40 | 40 | 40 |
| **Total** | | 100 | 100 | 100 | 100 | 100 | 100 | 100 | 100 | 100 | 100 |
| **Appearance** | | White emulsion-like | White emulsion-like | White emulsion-like | White emulsion-like | White emulsion-like | White emulsion-like | White emulsion-like | White emulsion-like | White emulsion-like | White emulsion-like |
| **Microscopic observation** | | No CAP crystals | No CAP crystals | No CAP crystals | No CAP crystals | No CAP crystals | No CAP crystals | No CAP crystals | No CAP crystals | No CAP crystals | No CAP crystals |

| **Formula** | | **A44** | | **A45** | | **A46** | | **A47** | | **A48** | |
|---|---|---|---|---|---|---|---|---|---|---|---|
| **Active ingredient** | **Capsaicin** | 8 | | 8 | | 8 | | 4 | | 8 | |
| **Water-soluble solvent** | **Ethanol** | 20 | | 20 | | 20 | | 20 | | 15 | |
| **Emulsifier** | **span 40** | 1 | | 2 | | 2 | | 2 | | 2 | |
| **Film-forming agent** | **PVA 17-88** | 7.5 | | / | | / | | 4 | | 7.5 | |
| | **PVA 20-88** | / | | 7.5 | | / | | / | | / | |
| | **PVA 24-88** | / | | / | | 7.5 | | / | | / | |
| **Thickening agent** | **Sorbitol** | 23.05 | | 22.05 | | 22.05 | | 29.55 | | 27.05 | |
| **Antioxidant** | **BHT** | 0.3 | | 0.3 | | 0.3 | | 0.3 | | 0.3 | |
| **Preservative** | **Methyl paraben** | 0.1 | | 0.1 | | 0.1 | | 0.1 | | 0.1 | |
| | **Propyl paraben** | 0.05 | | 0.05 | | 0.05 | | 0.05 | | 0.05 | |
| **Solvent** | **Purified water** | 40 | | 40 | | 40 | | 40 | | 40 | |
| **Total** | | 100 | | 100 | | 100 | | 100 | | 100 | |
| **Appearance** | | White emulsion-like | | White emulsion-like | | White emulsion-like | | White emulsion-like | | White emulsion-like | |
| **Microscopic observation** | | No CAP crystals | | No CAP crystals | | No CAP crystals | | No CAP crystals | | No CAP crystals | |

After capsaicin, ethanol and water form a liquid-liquid phase separation system, commonly used topical formulation excipients can be added to further develop cream products with different drug-loading capacities, with the maximum drug-loading capacity of capsaicin reaching up to 20%.

### Example 12. Preparation of Cream Compositions with Different Capsaicin Contents

According to the formula composition listed in Table 23, capsaicin was first dissolved in a water-soluble solvent, and then a fragrance agent, an antioxidant, and a preservative were added. The mixture was stirred for dissolution to obtain an oil phase. Separately, polyvinyl alcohol was dispersed in cold water, and then heated to 70 °C for dissolution to obtain an aqueous phase. The aqueous phase was added to the oil phase, and then an emulsifier and a film-forming auxiliary agent or thickening agent were added. The mixture was sheared at 70 °C and 10000 rpm for 5 min to prepare a cream.

**Table 23. Formulas of capsaicin creams with different drug-loading capacities and ethanol amounts**

| **Formula** | | **A34** | **A35** | **A36** | **A37** | **A38** | **A39** | **A40** | **A41** | **A42** | **A43** |
|---|---|---|---|---|---|---|---|---|---|---|---|
| **Active ingredient** | **Capsaicin** | 4.0 | 4.0 | 4.0 | 6.0 | 2.0 | 0.025 | 40 | 3.0 | 30 | / |
| **Water-soluble** | **Ethanol** | 20.0 | 0.0 | 30.0 | 20.0 | 20.0 | 20.0 | 20.0 | 20.0 | / | 20.0 |
| **solvent** | **DMI** | / | / | / | / | / | / | / | / | 20.0 | / |
| **Fragrance agent** | **Crystalline menthol** | 0.8 | 0.8 | 0.8 | 0.8 | 0.8 | 0.8 | 0.8 | 0.2 | 0.2 | 0.8 |
| **Antioxidant** | **BHT** | 0.3 | 0.3 | 0.3 | 0.3 | 0.3 | 0.3 | 0.3 | 0.3 | 0.3 | 0.3 |
| **Emulsifier** | **Span 40** | 2.0 | 2.0 | 2.0 | 2.0 | 2.0 | 2.0 | 2.0 | 2.0 | 2.0 | 2.0 |
| **Film-forming material** | **PVA 17-88** | 10.0 | 10.0 | 10.0 | 10.0 | 10.0 | 10.0 | 10.0 | / | / | 10.0 |
| | **PVA 05-88** | / | / | / | / | / | / | / | 15 | 15 | / |
| **Film-forming auxiliary agent** | **Anhydrous calcium hydrogen phosphate** | 18.7 | 18.7 | 7.75 | 18.7 | 18.7 | 19.7 | 28.75 | / | / | 18.7 |
| **Thickening agent** | **Sorbitol** | 10.0 | 10.0 | 10.0 | 8.0 | 12.0 | 14.025 | / | 17.35 | 17.35 | 10.0 |
| **Preservative** | **Methyl paraben** | 0.1 | 0.1 | 0.1 | 0.1 | 0.1 | 0.1 | 0.1 | 0.1 | 0.1 | 0.1 |
| | Propyl paraben | 0.05 | 0.05 | 0.05 | 0.05 | 0.05 | 0.05 | 0.05 | 0.05 | 0.05 | 0.05 |
| **Solvent** | **Purified water** | 34.05 | 54.05 | 35.0 | 34.05 | 34.05 | 34.0 | 34.0 | 42 | 42 | 38.05 |
| **Total** | | 100.0 | 100.0 | 100.0 | 100.0 | 100.0 | 100.0 | 100.0 | 100.0 | 100.0 | 100.0 |
| **Appearance** | | White emulsion-like | White emulsion-like | White emulsion-like | White emulsion-like | White emulsion-like | White emulsion-like | White emulsion-like | White emulsion-like | White emulsion-like | White emulsion-like |
| **Microscopic observation** | | No CAP crystals | A Large number of CAP crystals observed | No CAP crystals | No CAP crystals | No CAP crystals | No CAP crystals | No CAP crystals | No CAP crystals | No CAP crystals | No CAP crystals |

| **Formula** | | | | **A49** | | **A50** | | **A51** | | **A52** | |
|---|---|---|---|---|---|---|---|---|---|---|---|
| **Active ingredient** | | **Capsaicin** | | 4.0 | | 8.0 | | 15.0 | | 20.0 | |
| **Water-soluble solvent** | | **Ethanol** | | 20.0 | | 20.0 | | 20.0 | | 20.0 | |
| **Antioxidant** | | **BHT** | | 0.3 | | 0.3 | | 0.3 | | 0.3 | |
| **Emulsifier** | | **Span 40** | | 2.0 | | 2.0 | | 2.0 | | 2.0 | |
| **Film-forming material** | | **PVA 17-88** | | 7.5 | | 7.5 | | 7.5 | | 7.5 | |
| **Thickening agent** | | **Sorbitol** | | 26.05 | | 22.05 | | 15.05 | | 15.05 | |
| **Preservative** | | **Methyl paraben** | | 0.1 | | 0.1 | | 0.1 | | 0.1 | |
| | | **Propyl paraben** | | 0.05 | | 0.05 | | 0.05 | | 0.05 | |
| **Solvent** | | **Purified water** | | 40.0 | | 40.0 | | 40.0 | | 35.0 | |
| **Total** | | | | 100.0 | | 100.0 | | 100.0 | | 100.0 | |
| **Appearance** | | | | White emulsion-like | | White emulsion-like | | White emulsion-like | | White emulsion-like | |
| **Microscopic observation** | | | | No CAP crystals | | No CAP crystals | | No CAP crystals | | No CAP crystals | |

Microscopic observation of composition A35 showed the presence of capsaicin crystals, with uncontrollable quality. The quality of other emulsion compositions was stable.

### Example 13. Study on Stability of Capsaicin/Ethanol Composition Creams

Creams of formulas A34 and A40 were separately filled into ointment tubes, and the samples were left to stand at 5 °C, 25 °C, 30 °C, and 40 °C for testing. The growth of capsaicin dimer degradation impurities was investigated, and the results are shown in Table 24.

**Table 24. Growth of dimer impurities in cream compositions**

| Component | 5°C | 25°C | 30°C | 40°C |
|---|---|---|---|---|
| | wt % | wt % | wt % | wt % |
| A34-3M | 0.01 | 0.01 | 0.01 | 0.01 |
| A40-3M | 0.01 | 0.01 | 0.01 | 0.01 |

The cream products of the present disclosure exhibited stable quality, with dimer impurities being controllable.

### Example 14. Study on Film Performance

The film-forming performance and peelability of the compositions in Example 12 were evaluated, and the tensile strength and elongation at break of the films formed by the compositions in Example 12 were also evaluated. The measurement methods were as follows:
Evaluation of film-forming performance and peelability: 0.1 g of the composition was applied to a 1 cm² area of a latex glove. After overnight standing, the film-forming performance and peelability were investigated.

Measurement of tensile strength: A rectangular area of 12 × 3 cm was drawn, and 3.5 g of the composition was applied to ensure a uniform film thickness without bubbles or edge defects. After overnight standing, the film was peeled off and cut into pieces of the same size for tensile strength testing.

The measurement results are shown in Table 25. The results show that both the capsaicin-containing cream A34 and the blank cream A43 could form a film after application. In the peelability evaluation, the film formed by the capsaicin-containing cream A34 could be completely peeled off, while the film formed by the blank cream A43 was broken into 3 pieces during the peeling process. The mechanical performance (tensile strength) of the film formed by the capsaicin-containing cream A34 was superior to that formed by the blank cream.

In the cream of the present disclosure, the film-forming material PVA, the film-forming auxiliary agent anhydrous calcium hydrogen phosphate, and capsaicin exhibited a synergistic effect. The film-forming performance of the drug-containing composition with the addition of the film-forming material or the film-forming auxiliary agent was superior to that of the drug-free composition.

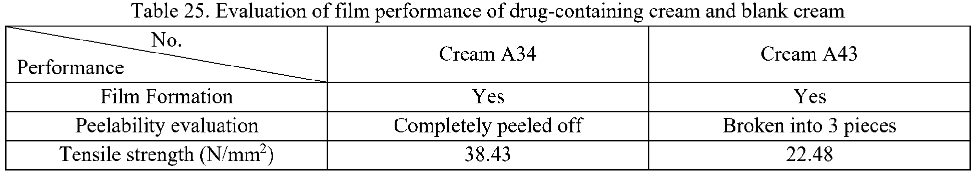

### Example 15. In Vitro Transdermal Study of Cream Compositions with Different Ethanol Contents

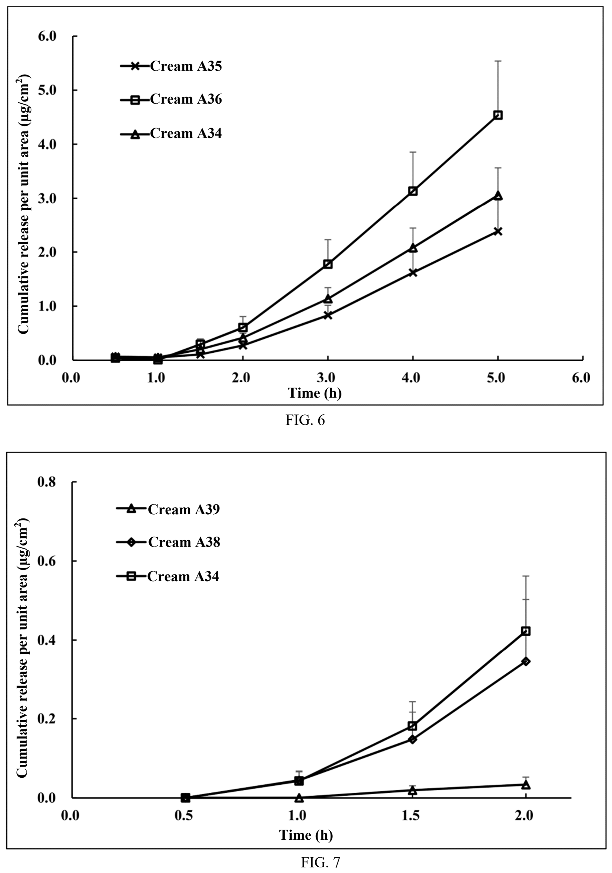

Pig skin was used as the *ex vivo* skin, and the Franz diffusion cell method was carried out to compare the *in vitro* transdermal performance of compositions A34 (4% capsaicin + 20% ethanol), A36 (4% capsaicin + 30% ethanol), and A35 (containing only 4% capsaicin). The receiving medium was 10% ethanol, the rotation speed was 200 rpm, the experimental temperature was 32 °C, and the drug dose was 300 mg. At 0.5 h, 1 h, 1.5 h, 2 h, 3 h, 4 h, and 6 h, samples of 1 mL were taken and then immediately supplemented with the same temperature and volume of medium. Each experimental group was conducted in parallel with four replicates. The *in vitro* transdermal results are shown in FIG. 6.

The results show that: both compositions A34 and A36 exhibited a certain transdermal rate, and the transdermal rate slightly increased with the ethanol concentration. Composition A35 did not contain ethanol, so capsaicin was precipitated as crystals in the cream. Although there was also a certain degree of transdermal absorption, the quality of the product was difficult to control due to the precipitated crystals. Therefore, the composition formed by capsaicin/water-soluble organic solvent can not only increase the transdermal absorption, but also greatly improve the stability of the cream, which is beneficial to the quality control of the product.

### Example 16. Comparison of In Vitro Transdermal Performance and Skin Drug Content of Cream Compositions With Different Capsaicin Contents

Pig skin was used as the *ex vivo* skin, and the Franz diffusion cell method was carried out to compare the *in vitro* transdermal performance of compositions A39 (0.025% capsaicin + 20% ethanol), A38 (2% capsaicin + 20% ethanol), and A34 (4% capsaicin + 20% ethanol). The receiving medium was 10% ethanol, the rotation speed was 200 rpm, the experimental temperature was 32 °C, and the drug dose was 300 mg. At 0.5 h, 1 h, 1.5 h, and 2 h, samples of 1 mL were taken and then immediately supplemented with the same temperature and volume of medium. Each experimental group was conducted in parallel with four replicates. After the experiment was completed, the pig skin was taken, wiped to remove the residual drug, rinsed with clean water for 2 min, and then wiped 10 times using PEG400 solvent-soaked cotton balls. The final wiping cotton ball was collected to detect the wiping residue. The pig skin was cut into small pieces, and then a 50% methanol solvent was added for shearing and extraction. The *in vitro* transdermal results are shown in FIG. 7, and the drug content in the *ex vivo* pig skin is shown in Table 26.

**Table 26. Drug content in skin from in vitro transdermal experiment**

| Drug content in skin at 1 h (µg, n = 4) | |
|---|---|
| A39 | 1.0±0.4 |
| A38 | 11.1±3.7 |
| A34 | 13.2±6.7 |

The results show that: the cream samples with different drug-loading capacities had different transdermal absorption rates. The transdermal amount and drug content in skin tissue for the 4% and 2% CAP cream samples were both higher than those for the commercially available over-the-counter 0.025% CAP cream. Therefore, high-concentration capsaicin creams can ensure the delivery of an effective drug dose to the skin. The drug content in skin tissue after 10 min of transdermal application of A49-A52 was comparable to, or exceeded, that of the commercially available formulation Qutenza^{®} after 1 h of transdermal application. High-concentration capsaicin creams can ensure the delivery of an effective drug dose to the skin. Moreover, the cream samples of A49-A52 exhibited good physical stability, remaining stable after centrifugation at 10000 rpm for 30 min, heat resistance testing at 60 °C for 24 h, and 3 freeze-thaw cycles. After topical administration, they exhibited excellent film-forming performance, and the films adhered well to the skin and were easy to peel off.

### Example 17. In Vitro Transdermal Comparison Between Cream Compositions and Commercially Available Formulation Qutenza^{®}

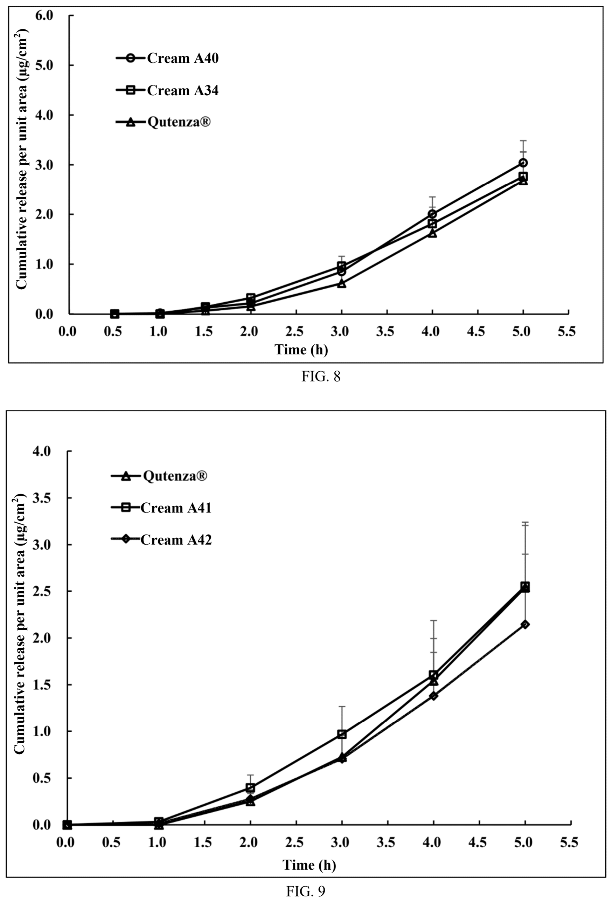

Pig skin was used as the *ex vivo* skin, and the Franz diffusion cell method was carried out to compare the *in vitro* transdermal performance of compositions A34 (4% capsaicin + 20% ethanol), A40 (4% capsaicin +20% ethanol) and A50 (8% capsaicin + 20% ethanol) and the commercially available formulation Qutenza^{®}. The receiving medium was 10% ethanol, the rotation speed was 200 rpm, the experimental temperature was 32 °C, and the drug dose was 300 mg. At 0.5 h, 1 h, 1.5 h, 2 h, 3 h, 4 h, and 5 h, samples of 1 mL were taken and then immediately supplemented with the same temperature and volume of medium. Each experimental group was conducted in parallel with four replicates. After the experiment was completed, the pig skin was taken, wiped to remove the residual drug, rinsed with clean water for 2 min, and then wiped 10 times using PEG400 solvent-soaked cotton balls. The final wiping cotton ball was collected to detect the wiping residue. The pig skin was cut into small pieces, and then a 50% methanol solvent was added for shearing and extraction. The *in vitro* transdermal results are shown in FIG. 8, and the drug content in the *ex vivo* pig skin is shown in Table 27.

**Table 27. Drug content in skin from in vitro transdermal experiment**

| Drug content in skin at 5 h (µg, n = 4) | |
|---|---|
| A34 | 9.1±1.9 |
| A40 | 12.0±1.4 |
| A50 | 13.36±1.45 |
| Qutenza^{®} | 13.1±3.2 |

The results show that: the *in vitro* transdermal experiment demonstrated comparable transdermal absorption rates for compositions A34, A40 and A50 and Qutenza^{®}, with substantially consistent drug content in skin tissue. Qutenza^{®}'s target site of action is located in the skin, so it can deliver a greater amount of effective medication to the skin within a short period of time, with a single application providing therapeutic efficacy lasting for 3 months.

### Example 18. In Vitro Transdermal Comparison Between Cream Compositions with Different Solvents and Commercially Available Formulation Qutenza^{®}

Pig skin was used as the *ex vivo* skin, and the Franz diffusion cell method was carried out to compare the *in vitro* transdermal performance of compositions A41 (3% capsaicin + 20% ethanol) and A42 (3% capsaicin + 20% DMI) and the commercially available formulation Qutenza^{®}. The receiving medium was 10% ethanol, the rotation speed was 200 rpm, and the experimental temperature was 32 °C.

IVPT experiment 1: By simulating the clinical administration dose, the designed drug dose was 100 mg (0.06 g/cm²). At 1 h, samples of 1 mL were taken. Each experimental group was conducted in parallel with five replicates. After the experiment was completed, the pig skin was taken, wiped to remove the residual drug, rinsed with clean water for 2 min, and then wiped 10 times using PEG400 solvent-soaked cotton balls. The final wiping cotton ball was collected to detect the wiping residue. The pig skin was cut into small pieces, and then a 50% methanol solvent was added for shearing and extraction. The drug content in the *ex vivo* pig skin is shown in Table 28.

**Table 28. Drug content in skin from in vitro transdermal experiment**

| Drug content in skin at 1 h (µg, n = 5) | |
|---|---|
| A41 | 3.88±0.9 |
| Qutenza^{®} | 2.32±0.1 |

IVPT experiment 2: By adopting an infinite dose, the designed drug dose was 300 mg (0.17 g/cm²). At 0.5 h, 1 h, 1.5 h, 2 h, 3 h, 4 h, and 5 h, samples of 1 mL were taken and then immediately supplemented with the same temperature and volume of medium. Each experimental group was conducted in parallel with five replicates. After the experiment was completed, the pig skin was taken, wiped to remove the residual drug, rinsed with clean water for 2 min, and then wiped 10 times using PEG400 solvent-soaked cotton balls. The final wiping cotton ball was collected to detect the wiping residue. The pig skin was cut into small pieces, and then a 50% methanol solvent was added for shearing and extraction. The *in vitro* transdermal results are shown in FIG. 9, and the drug content in the *ex vivo* pig skin is shown in Table 29.

**Table 29. Drug content in skin from in vitro transdermal experiment**

| Drug content in skin at 5 h (µg, n = 4) | |
|---|---|
| A41 | 11.1±3.8 |
| A42 | 12.1±4.5 |
| Qutenza^{®} | 12.7±3.3 |

The results show that: the *in vitro* transdermal experiment demonstrated that the drug content in skin tissue after simulated clinical administration of A41 and A42 cream compositions was higher than that of Qutenza^{®}, and their *in vitro* transdermal rates at the infinite dose were comparable to that of Qutenza^{®}. Since capsaicin's target site of action is located in the skin, it can deliver a greater amount of effective medication to the skin within a short period of time. Compared to Qutenza^{®}, the A41 and A42 creams are expected to achieve 3-month therapeutic efficacy with a shorter administration time in clinical practice.

### Example 19. Study on Irritation in Animals

Investigation of irritation of compositions disclosed herein:
According to the results of the toxicological study of Qutenza^{®} (Study no: 7215-113), when the administration area was 600 cm² (containing 384 mg of capsaicin), miniature pigs exhibited significant irritation reactions, making it impossible to continue the experiment. The protocol was adjusted to administer Demerol^{™} (meperidine) via intramuscular injection at a dose of 2 mg/kg prior to capsaicin administration, but irritation persisted. Therefore, the dose of Demerol^{™} was increased to 10 mg/kg.

The maximum human administration area of the creams disclosed herein was intended to be the same as that of Qutenza^{®}. The maximum dose stated in the Qutenza^{®} instructions is 4 patches, equivalent to 1120 cm². When used in miniature pigs (with a body weight of 20 kg), the corresponding application area is 588 cm², rounded to 600 cm². If the miniature pig weighs significantly less than 20 kg, the application area is calculated as 10% BSA.

The administration site area for all animals was 10% BSA (the administration site could be defined according to the actual condition of the animal). The calculated body surface area should be rounded to the nearest whole number, and the actual administration area should fall within 90%-110% of the theoretical administration area. The test sample was applied to the back and lateral abdomen of the animals using an appropriate scraper. Efforts were made to ensure a uniform and consistent thickness during application, with the administration time controlled within 3 min. If it is found that the drug is rubbed off during animal activities, the administration site can be covered with sterile gauze and further secured with an elastic bandage (the elastic bandage should not be too tight to avoid affecting the subsequent behavioral observations of the pigs). After the application was completed and the test sample formed a film, the animal was returned to the rearing cage. Before each administration, the administration site was cleaned with warm water, dried with gauze, and then subjected to drug application. Three hours after the administration, the film formed by the ointment was gently peeled off, and the administration site was wiped 2-3 times with 75% alcohol, then cleaned at least 3 times with clean water, and finally dried with gauze. The irritation results are shown in Table 30.

**Table 30. Irritation results**

| Group | Composition No. | Pretreatment* | Drug application area (cm²) | Dose (g) | Skin irritation score | Clinical observation result** | | | Tolerance evaluation |
|---|---|---|---|---|---|---|---|---|---|
| | | | | | | Severe reaction | Moderate reaction | Mild reaction | |
| 1 | A34 | Tolfedine was given 15 min before administration | 600 | 40 | 0*** | None | During the period of 1.3 h to 1.5 h after administration | Wall-rubbing once at 0.7 h after administration; shaking once at 1.8 h after administration | Tolerant |
| 2 | A40 | N/A | 220 | 18 | 0*** | None | None | None | Tolerant |

| | | | | | | | | | |
|---|---|---|---|---|---|---|---|---|---|
| *15 min before administration, Tolfedine was given via intramuscular injection at a dose of 2 mg/kg. **Evaluation criteria for clinical observation results: Moderate reaction symptoms: Low frequency of shaking and wall-rubbing. Mild reaction symptoms: Occasional occurrence of shaking and wall-rubbing. ***0 = No erythema. | | | | | | | | | |

According to the results listed in Table 30, it was unexpectedly found that under analgesic treatment with Tolfedine prior to administration, the A34 cream caused only moderate and mild irritation reactions during the administration period, which was evaluated as tolerable based on the irritation results. In contrast, when the A40 cream was administered to a 220 cm² area without analgesic treatment, it did not cause irritation reactions in the animals. This suggests that the compositions of the present disclosure can reduce capsaicin-induced irritation compared to Qutenza^{®}.

### Example 20. Study on Irritation in Volunteers

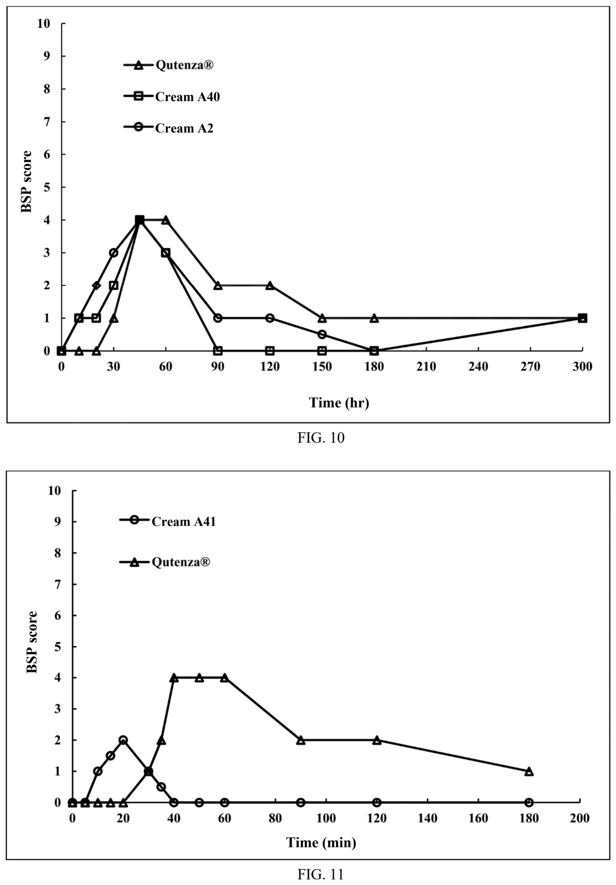

Cream A2, cream A40, and Qutenza^{®} were administered simultaneously to the arm of volunteer 1, with an administration area of 1.5 cm × 1.5 cm and an administration time of 45 min. A numerical pain scale was used to calculate BSP scores (0-10, higher values indicate greater pain), and the results are shown in FIG. 10. The BSP scores show that: mild pain could be felt 10 min after the administration of A40, and the pain increased over time, reaching its peak at the moment of removal, after which the pain decreased and essentially returned to normal after 1.5 h; mild pain could be felt 10 min after the administration of cream A2, and the pain increased over time, reaching its peak at the moment of removal, after which the pain slowly decreased and essentially returned to normal after 2.5 h; during the 45-min application of Qutenza^{®}, mild pain was only felt after 30 min, reaching its peak also at the moment of removal, but the intensity and duration of pain after removal were higher than those observed with A40. Both A40 and Qutenza^{®} caused mild irritation when the skin was exposed to hot water during bathing at 6 h.

A41 and Qutenza^{®} were administered simultaneously to the arm of volunteer 2, with an administration area of 2 cm × 2 cm and an administration time of 30 min. A numerical pain scale was used to calculate BSP scores (0-10, higher values indicate greater pain), and the results are shown in FIG. 11. The BSP scores show that: mild pain could be felt 10 min after the administration of A41, and the pain increased over time, with a maximum score of 2, and it essentially returned to normal after removal at 30 min; mild pain was only felt 30 min after the administration of Qutenza^{®}, reaching its peak after removal and lasting for 20 min, which was prolonged.

The embodiments of the present disclosure have been described above. However, the present disclosure is not limited to the embodiments described above. Any modification, equivalent replacement, improvement, and the like made without departing from the spirit and principle of the present disclosure shall fall within the claimed scope of the present disclosure.

## Claims

1. An emulsion composition of capsaicin, comprising:
a. capsaicin;
b. a water-soluble organic solvent;
c. water; and
d. an emulsifier.

2. The emulsion composition according to claim 1, wherein a mass percentage of capsaicin in the emulsion composition ranges from 0.01% to 20%, preferably 0.01% to 12%, preferably 0.25% to 12%, preferably 0.25% to 10%, preferably 0.25% to 8%, preferably 0.25% to 6%, preferably 1% to 6%, preferably 2% to 6%, and more preferably 2% to 4%.

3. The emulsion composition according to claim 1, wherein a mass ratio of capsaicin to the water-soluble organic solvent ranges from 1:30 to 1:0.1, preferably 1:15 to 1:0.5, and more preferably 1:10 to 1:1.

4. The emulsion composition according to claim 1, wherein in the emulsion composition, the three components of capsaicin, the water-soluble organic solvent, and water can form a liquid-liquid phase separation system.

5. The emulsion composition according to claim 1, wherein a mass ratio of water to the water-soluble organic solvent ranges from 0.2:1 to 10:1, preferably 0.2:1 to 5:1, preferably 0.5:1 to 3:1, and more preferably 0.5:1 to 2.5:1.

6. The emulsion composition according to any one of claims 1 to 5, wherein the water-soluble organic solvent is selected from one or more of methanol, ethanol, isopropanol, *tert*-butanol, diethylene glycol monoethyl ether, isosorbide dimethyl ether, acetonitrile, and triethanolamine;
the emulsifier is selected from one or more of a nonionic emulsifier, an ionic emulsifier, a natural emulsifier, and a polymer emulsifier, preferably selected from one or more of Span 40, Tween 20, Tween 80, glyceryl monostearate, poloxamer 188, polyoxyethylene hydrogenated castor oil, sodium dodecyl sulfate, sodium hexadecyl sulfate, sodium stearyl sulfate, triethanolamine lauryl sulfate, soybean phosphatidylcholine, egg-phosphatidylcholine, and polyvinyl alcohol;
preferably, a mass percentage of the emulsifier in the composition ranges from 0.1% to 10%, preferably 0.2% to 8%, and more preferably 0.5% to 5%.

7. The emulsion composition according to any one of claims 1 to 5, wherein the composition further comprises e. a film-forming material and/or a film-forming auxiliary agent and/or a thickening agent, preferably, the film-forming material is selected from one or more of polyvinyl alcohol, polyvinylpyrrolidone, methacrylic acid copolymer, vinylpyrrolidone/vinyl acetate copolymer, ethylcellulose, hydroxypropyl methylcellulose, and sodium carboxymethyl cellulose; the film-forming auxiliary agent is selected from anhydrous calcium hydrogen phosphate; preferably, a mass percentage of the film-forming material in the composition ranges from 0.01% to 25%, preferably 2% to 20%, and more preferably 5% to 15%; a mass percentage of the film-forming auxiliary agent in the composition ranges from 0.01% to 25%, preferably 2% to 20%, and preferably 5% to 15%; preferably, the thickening agent is selected from one or more of sorbitol, sucrose, and a film-forming material, and a mass percentage of the thickening agent in the composition ranges from 0.01% to 35%, preferably 0.01% to 30%, preferably 0.01% to 25%, preferably 2% to 20%, and more preferably 5% to 15%.

8. The emulsion composition according to claim 1, wherein the composition further comprises f. at least one pharmaceutically acceptable preservative, preferably, the preservative is selected from one or more of methyl paraben, ethyl paraben, propyl paraben, benzoic acid, potassium sorbate, sodium benzoate, calcium sorbate, benzalkonium bromide, benzalkonium chloride, o-phenylphenol, and chlorhexidine acetate; preferably, a mass percentage of the preservative in the composition ranges from 0.01% to 0.5%, preferably 0.05% to 2%, preferably 0.08% to 1%, preferably 0.1% to 0.5%, and more preferably 0.1% to 0.2%.

9. The emulsion composition according to claim 1, wherein the composition further comprises g. at least one pharmaceutically acceptable antioxidant, preferably, the antioxidant is selected from one or more of thioglycerol, sodium hydrogen sulfite, butylated hydroxytoluene, butylated hydroxyanisole, 2,6-di-*tert*-butyl-*p*-cresol, sodium sulfite, sodium metabisulfite, sodium thiosulfate, and vitamin C and vitamin E; preferably, a mass percentage of the antioxidant in the composition ranges from 0.01% to 5%, preferably 0.05% to 1%, and preferably 0.1% to 0.5%.

10. The emulsion composition according to any one of claims 1 to 9, wherein the composition is an emulsion or a cream, preferably a topical cream applied to the skin; preferably, the composition is an emulsion-based film.

11. A preparation method for the emulsion composition according to any one of claims 1 to 10, wherein the preparation method comprises: first dissolving capsaicin in the water-soluble organic solvent to obtain an oil phase, adding water to the oil phase, and then adding the emulsifier, followed by heating and shearing to prepare an emulsion;
or the preparation method comprises: first dissolving capsaicin in the water-soluble organic solvent to obtain an oil phase; then dissolving the film-forming material in water to obtain an aqueous phase; adding the aqueous phase to the oil phase, and then adding the emulsifier or the film-forming auxiliary agent to prepare an emulsion-based film;
preferably, the preparation method comprises the following steps:
a1. dissolving capsaicin in the water-soluble organic solvent, adding at least one pharmaceutically acceptable preservative if necessary, adding at least one pharmaceutically acceptable antioxidant if necessary, and heating for dissolution if necessary, to obtain an oil phase;
a2. adding the film-forming material or the film-forming auxiliary agent to water, and heating for dissolution if necessary, to obtain an aqueous phase; and
a3. mixing the aqueous phase, the oil phase, and the emulsifier, adding at least one pharmaceutically acceptable auxiliary agent if necessary, and
using a shearing device, such as a homogenizer or an emulsifying machine, for uniform mixing to obtain a sample;
or the preparation method comprises the following steps:
b1. dissolving capsaicin in the water-soluble organic solvent, and heating for dissolution if necessary, to obtain an oil phase;
b2. adding the film-forming material or the film-forming auxiliary agent to water, and heating for dissolution if necessary, to obtain an aqueous phase; and
b3. mixing the aqueous phase, the oil phase, and the emulsifier, adding at least one pharmaceutically acceptable preservative if necessary, adding
at least one pharmaceutically acceptable antioxidant if necessary, adding at least one pharmaceutically acceptable auxiliary agent if necessary, and using a shearing device, such as a homogenizer or an emulsifying machine, for uniform mixing to obtain a sample;
or the preparation method comprises the following steps:
c1. dissolving capsaicin in the water-soluble organic solvent, adding the emulsifier, and heating for dissolution if necessary, to obtain an oil phase;
c2. adding the film-forming material or the film-forming auxiliary agent to water, and heating for dissolution if necessary, to obtain an aqueous phase;
c3. mixing the aqueous phase and the oil phase, adding at least one pharmaceutically acceptable preservative if necessary, adding
at least one pharmaceutically acceptable antioxidant if necessary, and using a shearing device, such as a homogenizer or an emulsifying machine, for uniform mixing to obtain a sample; and
c4. further adding at least one pharmaceutically acceptable auxiliary agent to obtain a sample.

12. Use of the emulsion composition according to any one of claims 1 to 10 in the preparation of a medicament for treating pain, wherein the pain is neuropathic pain; preferably, the neuropathic pain is peripheral neuropathic pain or central neuropathic pain; preferably, the peripheral neuropathic pain is selected from painful diabetic peripheral neuropathy, postherpetic neuralgia, postoperative or post-traumatic peripheral neuropathic pain, drug-induced peripheral neuropathic pain, cancer pain, trigeminal neuralgia, and glossopharyngeal neuralgia.

13. Use of a water-soluble organic solvent as an oil phase in the preparation of an emulsion composition of capsaicin, wherein preferably, the water-soluble organic solvent is selected from one or more of methanol, ethanol, isopropanol, *tert*-butanol, diethylene glycol monoethyl ether, isosorbide dimethyl ether, acetonitrile, and triethanolamine.
